# EUROPEAN PATENT APPLICATION

(11) **EP 3 954 683 A1**
(43) Date of publication of application: **16.02.2022**
(21) Application number: 20798715.7
(22) Date of filing: 29.04.2020
(51) Int. Cl.: C07D 413/04, A61K 31/4725, A61K 9/16, A61P 19/00, A61P 29/00

(54) **PRODRUG OF CASPASE INHIBITOR**

(30) Priority: 30.04.2019 KR 20190051041
(71) Applicant: LG CHEM, LTD., Yeongdeungpo-gu, Seoul 07336 (KR)
(72) Inventor: CHOI, Sei Hyun, Seoul 07796 (KR); KIM, Sung Won, Seoul 07796 (KR); SONG, Jeong Uk, Seoul 07796 (KR); BAEK, Jae Uk, Seoul 07796 (KR); PARK, Hyun Seo, Seoul 07796 (KR); PARK, Ah Byeol, Seoul 07796 (KR); KIM, Jeong Ae, Seoul 07796 (KR); KANG, So Yeong, Seoul 07796 (KR); MOON, Hee Jeong, Seoul 07796 (KR)
(74) Representative: Hoffmann Eitle
(86) International application number: PCT/KR2020/005709
(87) International publication number: WO 2020/222541

(57) **Abstract**

The present invention relates to: an isooxazoline derivative having an ester moiety, the isoxazoline derivative being a prodrug of a caspase inhibitor, and a pharmaceutical composition containing same.

## Description

### TECHNICAL FIELD

The present invention relates to an isoxazoline derivative having ester moiety as a prodrug of caspase inhibitor and a pharmaceutical composition comprising the same.

### BACKGROUND ART

Caspases are a type of enzymes and are cysteine proteases that exist as an α2β2 tetramer. Caspase inhibitors interfere with the activity of these caspases, thereby regulating inflammation or apoptosis caused by the action of caspases. Diseases in which symptoms can be eliminated or alleviated by administration of these compounds include osteoarthritis, rheumatoid arthritis, degenerative arthritis, destructive bone disorder, hepatic diseases caused by hepatitis virus, acute hepatitis, hepatocirrhosis, brain damages caused by hepatitis virus, human fulminant liver failure, sepsis, organ transplantation rejection, ischemic cardiac disease, dementia, stroke, brain impairment due to AIDS, diabetes, gastric ulcer, etc.

Among compounds having various structures known as caspase inhibitors, isoxazoline derivatives were filed as Korean Patent Application Nos. 10-2004-0066726, 10-2006-0013107 and 10-2008-0025123. In addition, a prodrug of a caspase inhibitor based on an isoxazoline derivative was disclosed in International Publication No. WO 2007/015931 (Applicant: Vertex Pharmaceuticals Incorporated, USA).

### DISCLOSURE OF INVENTION

### TECHNICAL PROBLEM

The present invention is intended to improve bioavailability by developing a prodrug of an isoxazoline derivative having the structure of Formula 2 which is an effective inhibitor against caspase. In addition, the caspase inhibitor of Formula 2 has high solubility in water and high hydrophilicity, so it may be advantageous for the development of oral formulations, but there may be a disadvantage in the development of long-acting formulations. As such, the present invention is intended to develop a prodrug form of the caspase inhibitor of Formula 2 having hydrophobicity to be advantageous for long-acting formulations.

### SOLUTION TO PROBLEM

In order to achieve the above object, the present invention provides a compound of the following Formula 1, or a pharmaceutically acceptable salt or isomer thereof: 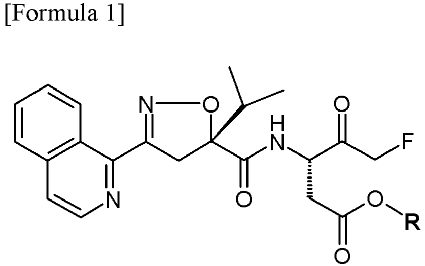 wherein
R represents alkyl, haloalkyl, cycloalkyl, cycloalkylalkyl, aryl, arylalkyl, heteroaryl, heteroarylalkyl, alkoxy or alkoxyalkyl, wherein the heteroaryl includes one or more heteroatoms selected from N, O and S;
wherein the alkyl, haloalkyl, cycloalkyl, cycloalkylalkyl, aryl, arylalkyl, heteroaryl, heteroarylalkyl or alkoxyalkyl is optionally substituted, and the substituent may be one or more selected from alkyl, halo, haloalkyl, cycloalkyl, hydroxy, acyl, amino, alkoxy, carboalkoxy, oxo, carboxy, carboxyamino, cyano, nitro, thiol, aryloxy, sulfoxy and guanido group;
provided that R is not tert-butyl.

The compound of Formula 1 according to the present invention may form a pharmaceutically acceptable salt. A pharmaceutically acceptable salt may include an acid-addition salt which is formed from an inorganic acid such as hydrochloric acid, sulfuric acid, nitric acid, phosphoric acid, hydrobromic acid and hydroiodic acid; an organic acid such as tartaric acid, formic acid, citric acid, acetic acid, trichloroacetic acid, trifluoroacetic acid, gluconic acid, benzoic acid, lactic acid, fumaric acid, maleic acid and salicylic acid; or sulfonic acid such as methanesulfonic acid, ethanesulfonic acid, benzenesulfonic acid and p-toluenesulfonic acid, which form non-toxic acid-addition salt including pharmaceutically acceptable anion. In addition, a pharmaceutically acceptable carboxylic acid salt includes the salt with alkali metal or alkali earth metal such as lithium, sodium, potassium, calcium and magnesium; salts with amino acid such as lysine, arginine and guanidine; an organic salt such as dicyclohexylamine, N-methyl-D-glucamine, tris(hydroxymethyl)methylamine, diethanolamine, choline and triethylamine. The compound of Formula 1 according to the present invention may be converted into their salts by conventional methods.

Meanwhile, since the compound of Formula 1 according to the present invention can have an asymmetric carbon center and asymmetric axis or plane, they can exist as E- or Z-isomer, R- or S-isomer, racemic mixtures or diastereoisomer mixtures and each diastereoisomer, all of which are within the scope of the present invention.

Herein, unless indicated otherwise, the term "the compound of Formula 1" is used to mean all the compounds of Formula 1, including the pharmaceutically acceptable salts and isomers thereof.

Herein, the following concepts defining the substituents are used to define the compound of Formula 1.

The term "halogen" or "halo" means fluoride (F), chlorine (Cl), bromine (Br) or iodine (I).

The term "alkyl" means straight or branched hydrocarbons, may include a single bond, a double bond or a triple bond, and is preferably C₁-C₁₈ alkyl. Examples of alkyl include, but are not limited to, methyl, ethyl, *n*-propyl, *i*-propyl, *n*-butyl, *i*-butyl, *tert*-butyl, pentyl, hexyl, heptyl, octyl, decyl, dodecyl, pentadecyl, octadecyl, acetylene, vinyl, trifluoromethyl and the like.

The term "cycloalkyl" means partially or fully saturated single or fused ring hydrocarbons, and is preferably C₃-C₁₀-cycloalkyl. Examples of cycloalkyl include, but are not limited to, cyclopropyl, cyclobutyl, cyclopentyl, cyclohexyl, cycloheptyl and the like.

Unless otherwise defined, the term "alkoxy" means alkyloxy having 1 to 10 carbon atoms.

The term "aryl" includes at least one ring having a conjugated pi (π) electron system, including-for example, monocyclic or fused-ring polycyclic (i.e., rings that share adjacent pairs of carbon atoms) groups. For example, the fused-ring polycyclic may include C₃-C₈ cycloalkyl ring fused with aryl. That is, unless otherwise defined herein, aryl is an aromatic monocyclic or polycyclic group having 5 to 15 carbon atoms, preferably 6 to 10 carbon atoms, including phenyl, naphthyl, dihydroindene, etc. For example, aryl may be C₅-C₁₂ aryl, preferably C₆-C₁₀ aryl.

The term "heteroaryl" means 3- to 12-membered, more preferably 5- to 10-membered aromatic hydrocarbons which form a single or fused ring-which may be fused with benzo or C₃-C₈ cycloalkyl-including one or more heteroatoms selected from N, O and S as a ring member. Examples of heteroaryl include, but are not limited to, pyridinyl, pyrimidinyl, pyridazinyl, pyrazinyl, oxadiazolyl, isoxadiazolyl, tetrazolyl, triazolyl, indolyl, indazolyl, isoxazolyl, oxazolyl, thiazolyl, isothiazolyl, furanyl, benzofuranyl, imidazolyl, thiophenyl, benzthiazole, benzimidazole, quinolinyl, indolinyl, 1,2,3,4-tetrahydroisoquinolyl, 3,4-dihydroisoquinolinyl, thiazolopyridyl, 2,3-dihydrobenzofuran, 2,3-dihydrothiophene, 2,3-dihydroindole, benzo[1,3]dioxin, chroman, thiochroman, 1,2,3,4-tetrahydroquinoline, 4H-benzo[1,3]dioxin, 2,3-dihydrobenzo[1,4]-dioxin, 6,7-dihydro-5H-cyclopenta[d]pyrimidine and the like.

Cycloalkyl-alkyl, aryl-alkyl, heteroaryl-alkyl and alkoxy-alkyl mean groups which are formed by the combination of the above-mentioned cycloalkyl, aryl, heteroryl, alkoxy and/or alkyl. Examples include, but are not limited to, benzyl, thiophenemethyl, pyrimidinemethyl and the like.

According to one embodiment of the present invention, R may represent C₁-₂₀ alkyl, C₃-₁₀ cycloalkyl, C₃-₁₀ cycloalkyl-C₁-₆ alkyl, C₆-C₁₀ aryl, C₆-C₁₀ aryl-C₁-₆ alkyl, 3- to 10-membered heteroaryl, 3- to 10-membered heteroaryl-C₁-₆ alkyl, halo-C₁-₆ alkyl or C₁-₆ alkoxy-C₁-₆ alkyl, wherein the heteroaryl may include 1 to 4 heteroatoms selected from N, O and S, but is not limited thereto.

According to one embodiment of the present invention, R may represent C₁-₁₈ alkyl, C₃-₆ cycloalkyl, C₃-₆ cycloalkyl-C₁-₃ alkyl, C₆-C₁₀ aryl, C₆-C₁₀ aryl-C₁-₃ alkyl, 4- to 6-membered heteroaryl-C₁-₃ alkyl, halo-C₁-₃ alkyl or C₁-₃ alkoxy-C₁-₃ alkyl, wherein the heteroaryl may include 1 or 2 heteroatoms selected from N, O and S, and the substituent may be alkyl, halo, alkoxy or oxo, but is not limited thereto.

Representative compounds of Formula 1 according to the present invention include, but are not limited to, the following compounds:
methyl (S)-5-fluoro-3-((R)-5-isopropyl-3-(isoquinolin-1-yl)-4,5-dihydroisoxazole-5-carboxamido)-4-oxopentanoate;
ethyl (S)-5-fluoro-3-((R)-5-isopropyl-3-(isoquinolin-1-yl)-4,5-dihydroisoxazole-5-carboxamido)-4-oxopentanoate;
propyl (S)-5-fluoro-3-((R)-5-isopropyl-3-(isoquinolin-1-yl)-4,5-dihydroisoxazole-5-carboxamido)-4-oxopentanoate;
butyl (S)-5-fluoro-3-((R)-5-isopropyl-3-(isoquinolin-1-yl)-4,5-dihydroisoxazole-5-carboxamido)-4-oxopentanoate;
isobutyl (S)-5-fluoro-3-((R)-5-isopropyl-3-(isoquinolin-1-yl)-4,5-dihydroisoxazole-5-carboxamido)-4-oxopentanoate;
isopentyl (S)-5-fluoro-3-((R)-5-isopropyl-3-(isoquinolin-1-yl)-4,5-dihydroisoxazole-5-carboxamido)-4-oxopentanoate;
pentyl (S)-5-fluoro-3-((R)-5-isopropyl-3-(isoquinolin-1-yl)-4,5-dihydroisoxazole-5-carboxamido)-4-oxopentanoate;
hexyl (S)-5-fluoro-3-((R)-5-isopropyl-3-(isoquinolin-1-yl)-4,5-dihydroisoxazole-5-carboxamido)-4-oxopentanoate;
heptyl (S)-5-fluoro-3-((R)-5-isopropyl-3-(isoquinolin-1-yl)-4,5-dihydroisoxazole-5-carboxamido)-4-oxopentanoate;
octyl (S)-5-fluoro-3-((R)-5-isopropyl-3-(isoquinolin-1-yl)-4,5-dihydroisoxazole-5-carboxamido)-4-oxopentanoate;
decyl (S)-5-fluoro-3-((R)-5-isopropyl-3-(isoquinolin-1-yl)-4,5-dihydroisoxazole-5-carboxamido)-4-oxopentanoate;
dodecyl (S)-5-fluoro-3-((R)-5-isopropyl-3-(isoquinolin-1-yl)-4,5-dihydroisoxazole-5-carboxamido)-4-oxopentanoate;
pentadecyl (S)-5-fluoro-3-((R)-5-isopropyl-3-(isoquinolin-1-yl)-4,5-dihydroisoxazole-5-carboxamido)-4-oxopentanoate;
octadecyl (S)-5-fluoro-3-((R)-5-isopropyl-3-(isoquinolin-1-yl)-4,5-dihydroisoxazole-5-carboxamido)-4-oxopentanoate;
(9E, 12E)-octadeca-9,12-dien-1-yl(S)-5-fluoro-3-((R)-5-isopropyl-3-(isoquinolin-1-yl)-4,5-dihydroisoxazole-5-carboxamido)-4-oxopentanoate;
cyclopropylmethyl (S)-5-fluoro-3-((R)-5-isopropyl-3-(isoquinolin-1-yl)-4,5-dihydroisoxazole-5-carboxamido)-4-oxopentanoate;
cyclobutylmethyl (S)-5-fluoro-3 -((R)-5 -isopropyl-3 -(isoquinolin-1-yl)-4, 5-dihydroisoxazole-5-carboxamido)-4-oxopentanoate;
cyclopentylmethyl (S)-5-fluoro-3-((R)-5-isopropyl-3-(isoquinolin-1-yl)-4,5-dihydroisoxazole-5-carboxamido)-4-oxopentanoate;
allyl (S)-5-fluoro-3-((R)-5-isopropyl-3-(isoquinolin-1-yl)-4,5-dihydroisoxazole-5-carboxamido)-4-oxopentanoate;
isopropyl (S)-5-fluoro-3-((R)-5-isopropyl-3-(isoquinolin-1-yl)-4,5-dihydroisoxazole-5-carboxamido)-4-oxopentanoate;
penta-3-yl (S)-5-fluoro-3-((R)-5-isopropyl-3-(isoquinolin-1-yl)-4,5 dihydroisoxazole-5-carboxamido)-4-oxopentanoate;
sec-butyl (S)-5-fluoro-3-((R)-5-isopropyl-3-(isoquinolin-1-yl)-4,5-dihydroisoxazole-5-carboxamido)-4-oxopentanoate;
pentan-2-yl (S)-5-fluoro-3-((R)-5-isopropyl-3-(isoquinolin-1-yl)-4,5-dihydroisoxazole-5-carboxamido)-4-oxopentanoate;
heptan-2-yl (S)-5-fluoro-3-((R)-5-isopropyl-3-(isoquinolin-1-yl)-4,5-dihydroisoxazole-5-carboxamido)-4-oxopentanoate;
cyclopentyl (S)-5-fluoro-3-((R)-5-isopropyl-3-(isoquinolin-1-yl)-4,5-dihydroisoxazole-5-carboxamido)-4-oxopentanoate;
cyclohexyl (S)-5-fluoro-3-((R)-5-isopropyl-3-(isoquinolin-1-yl)-4,5-dihydroisoxazole-5-carboxamido)-4-oxopentanoate
benzyl (S)-5-fluoro-3-((R)-5-isopropyl-3-(isoquinolin-1-yl)-4,5-dihydroisoxazole-5-carboxamido)-4-oxopentanoate;
(5-methyl-2-oxo-1,3-dioxol-4-yl)methyl (S)-5-fluoro-3-((R)-5-isopropyl-3-(isoquinolin-1-yl)-4,5-dihydroisoxazole-5-carboxamido)-4-oxopentanoate;
2-methoxyphenyl (S)-5-fluoro-3-((R)-5-isopropyl-3-(isoquinolin-1-yl)-4,5-dihydroisoxazole-5-carboxamido)-4-oxopentanoate;
2,3-dihydro-1*H*-inden-5-yl (S)-5-fluoro-3-((R)-5-isopropyl-3-(isoquinolin-1-yl)-4,5-dihydroisoxazole-5-carboxamido)-4-oxopentanoate;
naphthalen-1-yl (S)-5-fluoro-3-((R)-5-isopropyl-3-(isoquinolin-1-yl)-4,5-dihydroisoxazole-5-carboxamido)-4-oxopentanoate;
phenyl (S)-5-fluoro-3-((R)-5-isopropyl-3-(isoquinolin-1-yl)-4,5-dihydroisoxazole-5-carboxamido)-4-oxopentanoate;
naphthalen-1-ylmethyl (S)-5-fluoro-3-((R)-5-isopropyl-3-(isoquinolin-1-yl)-4,5-dihydroisoxazole-5-carboxamido)-4-oxopentanoate;
2,2,2-trifluoroethyl (S)-5-fluoro-3-((R)-5-isopropyl-3-(isoquinolin-1-yl)-4,5-dihydroisoxazole-5-carboxamido)-4-oxopentanoate;
2-methoxyethyl (S)-5-fluoro-3-((R)-5-isopropyl-3-(isoquinolin-1-yl)-4,5-dihydroisoxazole-5-carboxamido)-4-oxopentanoate;
2-fluoroethyl (S)-5-fluoro-3-((R)-5-isopropyl-3-(isoquinolin-1-yl)-4,5-dihydroisoxazole-5-carboxamido)-4-oxopentanoate;
neopentyl (S)-5-fluoro-3-((R)-5-isopropyl-3-(isoquinolin-1-yl)-4,5-dihydroisoxazole-5-carboxamido)-4-oxopentanoate;
thiophen-2-ylmethyl (S)-5-fluoro-3-((R)-5-isopropyl-3-(isoquinolin-1-yl)-4,5-dihydroisoxazole-5-carboxamido)-4-oxopentanoate;
thiophen-3-ylmethyl (S)-5-fluoro-3-((R)-5-isopropyl-3-(isoquinolin-1-yl)-4,5-dihydroisoxazole-5-carboxamido)-4-oxopentanoate; and
furan-3-ylmethyl (S)-5-fluoro-3-((R)-5-isopropyl-3-(isoquinolin-1-yl)-4,5-dihydroisoxazole-5-carboxamido)-4-oxopentanoate.

The terms and abbreviations used herein retain their original meanings unless indicated otherwise.

The present invention also provides a method for preparing the compound of Formula 1. Hereinafter, the method for preparing the compound of Formula 1 is explained based on exemplary reactions in order to illustrate the present invention. However, a person skilled in the art could prepare the compound of Formula 1 by various methods based on the structure of Formula 1, and such methods should be interpreted as being within the scope of the present invention. That is, the compound of Formula 1 may be prepared by the methods described herein or by combining various methods disclosed in the prior art, which should be interpreted as being within the scope of the present invention. Accordingly, a method for preparing the compound of Formula 1 is not limited to the following methods.

The compound of Formula 1 of the present invention may be prepared from the compound of Formula 2 according to the method of the following Reaction Scheme 1. The compound of Formula 1-which is a prodrug-may be synthesized by the use of the compound of Formula 2 and oxalyl chloride, dimethyl formamide (DMF), alcohol and dichloromethane (DCM) solvent, or may be synthesized by the use of the compound of Formula 2 and an alkyl halide, potassium carbonate and dimethyl formamide solvent, or may be synthesized by the use of the compound of Formula 2 and EDC (3-ethyliminomethyleneamino-N,N-dimethylpropan-1-amine) or EDCI (N-ethyl-N'-(3-dimethylaminopropyl)carbodiimide hydrochloride), HOBt (1-hydroxybenzotriazole), triethylamine (Et3N), alkyl alcohol and dichloromethane solvent.

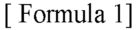

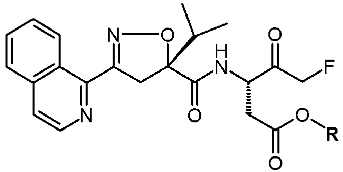

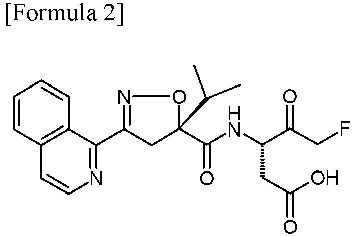

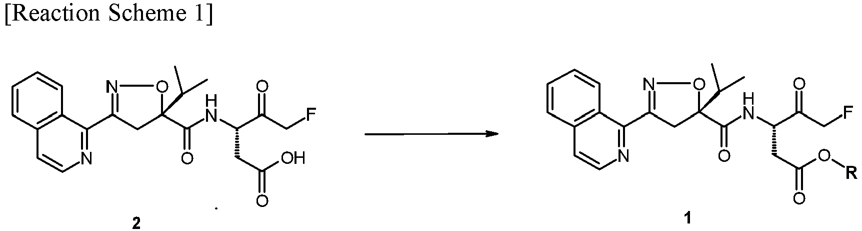

A compound not specifically described in the preparation method of the present specification is a known compound or a compound that can be easily synthesized from a known compound by a known synthesis method or a similar method.

The compound of Formula 1 obtained by the above methods can be separated or purified from the reaction products by conventional methods such as recrystallization, ionospheresis, silica gel column chromatography or ion-exchange chromatography.

As explained above, the compounds according to the present invention, starting materials or intermediates for the preparation thereof can be prepared by a variety of methods, which should be interpreted as being within the scope of the present invention in respect to the preparation of the compound of Formula 1.

The compound of Formula 1 according to the present invention can be used as a prodrug of caspase inhibitor. Accordingly, the present invention provides a pharmaceutical composition for the prevention or treatment of inflammation or apoptosis comprising the compound of Formula 1, or a pharmaceutically acceptable salt or isomer thereof as an active ingredient, together with a pharmaceutically acceptable carrier.

Exemplary diseases that can be prevented or treated by the pharmaceutical composition according to the present invention include, but are not limited to, those selected from the group consisting of apoptosis-associated diseases, inflammatory diseases, osteoarthritis, rheumatoid arthritis, degenerative arthritis and destructive bone disorders.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethyl sulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt or isomer thereof-is mixed with selected pharmaceutically acceptable carriers considering the dosage form to be prepared. For example, the pharmaceutical composition of the present invention can be formulated as injections, oral preparations and the like, as needed.

The pharmaceutical composition of the present invention may be formulated in oral dosage form, injection form or patch form, but may not be limited thereto.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like.

In the case of parenteral formulations, sterilized water is used usually and other ingredient(s) such as a dissolution adjuvant may also be comprised. Injection formulations, for example, sterilized aqueous- or oil-based suspension for injection may be prepared according to known techniques by using appropriate dispersing agent, wetting agent or suspending agent. The solvents useful for this purpose include water, ringer solution and isotonic NaCl solution, and sterilized, immobilized oils are also used as a solvent or a suspending medium conventionally. Any non-irritant immobilized oils including mono- and di-glycerides may be used for this purpose, and fatty acids such as an oleic acid may be used for an injection formulation. In the case of percutaneous formulations, a penetration-enhancing agent and/or a suitable wetting agent may be used as a carrier, optionally in combination with suitable non-irritant additive(s) to the skin. As such additives, those helpful in enhancing the administration through the skin and/or preparing the desired composition may be selected. The percutaneous formulation may be administered in various ways-for example, such as a transdermal patch, a spot-on treatment or an ointment.

The compound or pharmaceutical composition comprising the same according to the present invention can be administered in combination with other drugs-for example, other caspase inhibitors and/or caspase inhibitor prodrugs.

The dose of the compound of Formula 1 according to the present invention is determined by a physician's prescription considering the patient's body weight, age, and specific condition and seriousness of the disease. When the compound of the present invention is administered for clinical purposes, the total daily dose to be administered to the host in a single dose or in separate doses is preferably in the range of about 5 to 500 mg/kg of body weight, but the specific dose level for a specific patient may vary depending on the patient's weight, sex, health status, diet, drug administration time, administration method, excretion rate, drug mixture, disease severity, etc.

Herein, the term "treatment" is used to mean deterring, delaying or ameliorating the progress of diseases in a subject exhibiting symptoms of diseases.

According to one embodiment of the present invention, the pharmaceutical composition may comprise a microsphere comprising the compound of Formula 1, or a pharmaceutically acceptable salt or isomer, and a biocompatible polymer, but is not limited thereto.

For example, the biocompatible polymer may be selected from polylactide, polyglycolide, polylactide-glycolide copolymer, poly(lactide-co-glycolide)glucose, polycaprolactone, gelatin and hyaluronate, and preferably polyglycolide, polylactide or polylactide-glycolide copolymer (PLGA).

According to one embodiment of the present invention, the biocompatible polymer may be a polylactide-glycolide copolymer (PLGA) having a molar ratio of lactide to glycolide of 10:90 to 90:10, but is not limited thereto. For example, the molar ratio may be preferably 50:50 to 75:25.

According to one embodiment of the present invention, the weight ratio of the compound of Formula 1, or a pharmaceutically acceptable salt or isomer thereof, and the biocompatible polymer in the microsphere may be 1:100 to 70:100, but is not limited thereto. Preferably, the weight ratio of the compound of Formula 1, or a pharmaceutically acceptable salt or isomer thereof, and the biocompatible polymer may be 1:100 to 17:100. If the weight ratio is lower or higher than the above range, the drug may not be properly encapsulated into the microspheres or a problem of aggregation of the microspheres may occur. For example, the compound of Formula 1, or a pharmaceutically acceptable salt or isomer thereof in the microspheres may be included in a weight ratio of 5% or more and less than 30% compared to the biocompatible polymer, preferably a weight ratio of 10% or more and less than 17%, and more preferably a weight ratio of about 16.7%, but is not limited thereto.

For example, the molecular weight range of the polylactide-glycolide copolymer may be about 1 to 1,000 kDa, preferably about 30 to 150 kDa, and more preferably about 38 to 54 kDa, but is not limited thereto.

For example, an end group of the polylactide-glycolide copolymer may be an ester or an acid, preferably an ester, but is not limited thereto.

For example, the weight ratio of the solid (drug and PLGA) to the solvent used in preparing the microspheres may be about 5% to 40%, preferably about 10% to 20%, and more preferably about 10%, but is not limited thereto.

For example, the diameter of the microspheres may be about 1 to 250 µm, preferably about 20 to 100 µm, and more preferably about 30 to 70 µm, but is not limited thereto.

Solvents useful for the preparation of the microspheres may be selected from dichloromethane, dimethylsulfoxide, dimethylformamide, acetic acid, hydrochloric acid, methanol, ethanol, acetone, ethanol, chloroform, acetonitrile, N-methyl-2-pyrrolidone, tetrahydrofuran, methyl ethyl ketone, propyl acetate, ethyl acetate and methyl acetate.

In the microsphere preparation step of the present invention, the removal of organic solvent may be carried out by applying any conventional solvent removal method-for example, solvent extraction and stirring, heating, solvent evaporation such as nitrogen purge (N₂ purge), etc.

### ADVANTAGEOUS EFFECTS OF INVENTION

The present invention relates to a novel compound having the structure of Formula 1, which is a prodrug of an isoxazoline derivative-which is a caspase inhibitor-having the structure of Formula 2. That is, the compound of Formula 1 acts as a prodrug of a caspase inhibitor. The prodrug compound having the structure of Formula 1 is converted into the active form of the caspase inhibitor of Formula 2 by an esterase isoenzyme in the body. These prodrug compounds have advantages over the caspase inhibitor of Formula 2 in terms of pharmacokinetics. Specifically, the prodrug compound of Formula 1 has increased drug durability compared to the caspase inhibitor of Formula 2. In addition, because the prodrug compound of Formula 1 can be converted into the caspase inhibitor of Formula 2 by a degrading enzyme in the human body and has hydrophobicity itself, it may be suitable for a long-acting formulation.

### BRIEF DESCRIPTION OF DRAWINGS

Figure 1 is a graph showing the conversion of a caspase prodrug into an active form of a caspase inhibitor by hydrolase in rat plasma.
Figure 2 is a graph showing the average concentration profile of the drug in the joint of a dog administered with a caspase prodrug.
Figure 3 is an image of the appearance of PLGA microspheres encapsulated with caspase prodrugs observed with a scanning electron microscope.
Figure 4 is an image of the properties of PLGA microspheres prepared by encapsulating a caspase inhibitor observed with a scanning electron microscope.
Figure 5 is an image of observing the properties of PLGA microspheres prepared by varying the weight ratio of a caspase prodrug and a polymer.
Figure 6 is an *in vitro* dissolution graph of PLGA microspheres encapsulated with caspase prodrugs in PBS and joint synovial fluid.
Figure 7 is an *in vitro* dissolution graph of PLGA microspheres prepared by varying the molar ratio of lactide to glycolide.
Figure 8 is photographs of observing the properties of microspheres during the dissolution test of PLGA microspheres prepared by varying the molar ratio of lactide to glycolide.
Figure 9 is a graph measuring the molecular weight change according to the progress of the dissolution test of microspheres.
Figure 10 is a graph showing the measurement of the concentration of caspase inhibitor in joint synovial fluid according to intra-articular administration of PLGA microspheres prepared according to one embodiment of the present invention.

### MODE FOR THE INVENTION

Hereinafter, the present invention will be described in more detail through preparation examples and examples. However, these examples are only illustrative, and the scope of the present invention is not limited thereto.

### Example 1: Methyl (S)-5-fluoro-3-((R)-5-isopropyl-3-(isoguinolin-1-yl)-4,5-dihydroisoxazole-5-carboxamido)-4-oxopentanoate

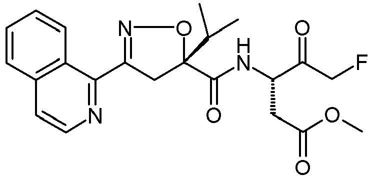

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and anhydrous methanol (2.0 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in isopropanol to obtain 2.4 g (yield: 46%) of the title compound.

¹H NMR (400 MHz, CDCl₃) δ 9.13 (d, 1H), 8.56 (d, 1H), 7.87 (d, 1H), 7.75-7.67 (m, 4H), 5.21 (m, 2H), 4.95 (m, 1H), 4.05 (m, 2H), 4.01 (d, 1H), 3.82 (d, 1H), 3.59 (s, 3H), 3.01 (dd, 2H), 2.41 (m, 1H), 1.12 (dd, 6H)

### Example 2: Ethyl (S)-5-fluoro-3-((R)-5-isopropyl-3-(isoquinolin-1-yl)-4,5-dihydroisoxazole-5-carboxamido)-4-oxopentanoate

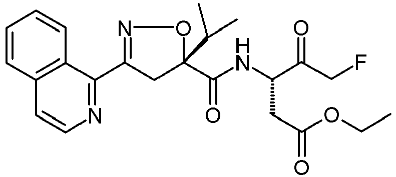

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and anhydrous ethanol (2.8 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:2 mixture of ethanol and hexane (EtOH: hexane=1:2) to obtain 4.1 g (yield: 76%) of the title compound.

¹H NMR (400 MHz, CDCl₃) δ 9.13 (d, 1H), 8.56 (d, 1H), 7.87 (d, 1H), 7.75-7.67 (m, 4H), 5.21 (m, 2H), 4.95 (m, 1H), 4.07 (m, 2H), 4.04 (d, 1H), 3.82 (d, 1H), 3.01 (dd, 2H), 2.41 (m, 1H), 1.12 (dd, 6H)

### Example 3: Propyl (S)-5-fluoro-3-((R)-5-isopropyl-3-(isoquinolin-1-yl)-4,5-dihydroisoxazole-5-carboxamido)-4-oxopentanoate

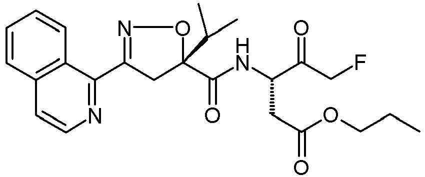

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and propanol (3.6 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.2 g (yield: 76%) of the title compound.

¹H NMR (400 MHz, CDCl₃) δ 9.14 (d, 1H), 8.56 (d, 1H), 7.87 (d, 1H), 7.75-7.67 (m, 4H), 5.21 (m, 2H), 4.95 (m, 1H), 4.05 (d, 1H), 3.94 (m, 2H), 3.82 (d, 1H), 3.01 (dd, 2H), 2.41 (m, 1H), 1.54 (m, 2H), 1.11 (dd, 6H), 0.80 (t, 3H)

### Example 4: Butyl (S)-5-fluoro-3-((R)-5-isopropyl-3-(isoquinolin-1-yl)-4,5-dihydroisoxazole-5-carboxamido)-4-oxopentanoate

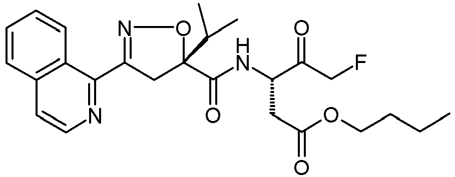

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and butanol (6.0 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.1 g (yield: 73%) of the title compound.

¹H NMR (400 MHz, CDCl₃) δ 9.15 (d, 1H), 8.56 (d, 1H), 7.88 (d, 1H), 7.75-7.67 (m, 4H), 5.21 (m, 2H), 4.95 (m, 1H), 4.04 (d, 1H), 3.95 (m, 2H), 3.82 (d, 1H), 3.01 (dd, 2H), 2.41 (m, 1H), 1.51 (m, 2H), 1.24 (m, 2H), 1.11 (dd, 6H), 0.81 (t, 3H)

### Example 5: Isobutyl (S)-5-fluoro-3-((R)-5-isopropyl-3-(isoauinolin-1-yl)-4,5-dihydroisoxazole-5-carboxamido)-4-oxopentanoate

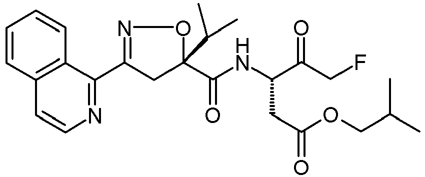

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and isobutanol (3.7 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 3.7 g (yield: 66%) of the title compound.

¹H NMR (400 MHz, CDCl₃) δ 9.14 (d, 1H), 8.55 (d, 1H), 7.88 (d, 1H), 7.75-7.67 (m, 4H), 5.20 (m, 2H), 4.95 (m, 1H), 4.03 (d, 1H), 3.81 (d, 1H), 3.77 (m, 2H), 3.01 (dd, 2H), 2.41 (m, 1H), 1.78 (m, 2H), 1.11 (dd, 6H), 0.77 (d, 6H)

### Example 6: Isopentyl (S)-5-fluoro-3-((R)-5-iso pro pyl-3-(isoquinolin-1-yl)-4,5-dihydroisoxazole-5-carboxamido)-4-oxopentanoate

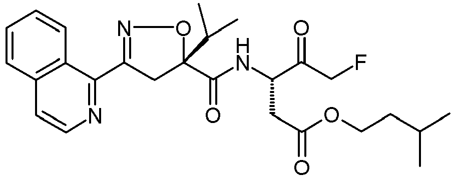

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving dichloromethane (50 mL), the temperature of the mixture was adjusted to 5°C, and anhydrous isopentanol (5.3 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.6 g (yield: 72%) of the title compound.

¹H NMR (400 MHz, CDCl₃) δ 9.13 (d, 1H), 8.55 (d, 1H), 7.88 (d, 1H), 7.75-7.67 (m, 4H), 5.20 (m, 2H), 4.95 (m, 1H), 4.03 (d, 1H), 3.99 (m, 2H), 3.82 (d, 1H), 3.01 (dd, 2H), 2.41 (m, 1H), 1.53 (m, 1H), 1.39 (m, 2H), 1.11 (dd, 6H), 0.79 (d, 6H)

### Example 7: Pentyl (S)-5-fluoro-3-((R)-5-isopropyl-3-(isoquinolin-1-yl)-4,5-dihydroisoxazole-5-carboxamido)-4-oxopentanoate

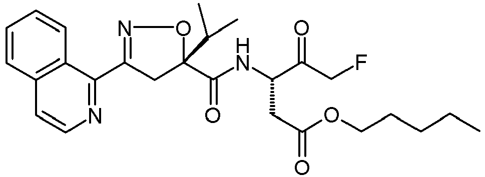

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and pentanol (2.6 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:10 mixture of ethanol and hexane (EtOH: hexane=1:10) to obtain 2.1 g (yield: 36%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.04 (d, 1H), 3.97 (m, 2H), 3.82 (d, 1H), 3.01 (dd, 2H), 2.41 (m, 1H), 1.51 (m, 2H), 1.23 (m, 4H), 1.11 (dd, 6H), 0.83 (t, 3H)

### Example 8: Hexyl (S)-5-fluoro-3-((R)-5-isopropyl-3-(isoguinolin-1-yl)-4,5-dihydroisoxazole-5-carboxamido)-4-oxopentanoate

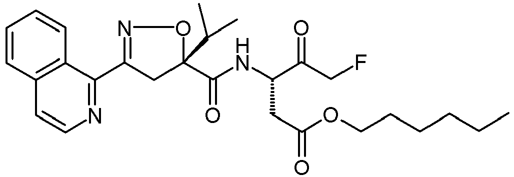

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and hexanol (6.0 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.4 g (yield: 73%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.04 (d, 1H), 3.97 (m, 2H), 3.82 (d, 1H), 3.01 (dd, 2H), 2.41 (m, 1H), 1.50 (m, 2H), 1.23 (m, 6H), 1.11 (dd, 6H), 0.84 (t, 3H)

### Example 9: Heptyl (S)-5-fluoro-3-((R)-5-isopropyl-3-(isoquinolin-1-yl)-4,5-dihydroisoxazole-5-carboxamido)-4-oxopentanoate

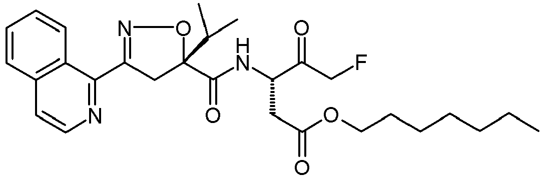

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and anhydrous heptanol (6.8 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.9 g (yield: 79%) of the title compound.

¹H NMR (400 MHz, CDCl₃) δ 9.14 (d, 1H), 8.56 (d, 1H), 7.88 (d, 1H), 7.75-7.67 (m, 4H), 5.21 (m, 2H), 4.95 (m, 1H), 4.04 (d, 1H), 3.98 (m, 2H), 3.82 (d, 1H), 3.01 (dd, 2H), 2.41 (m, 1H), 1.49 (m, 2H), 1.20 (m, 8H), 1.11 (dd, 6H), 0.84 (t, 3H)

### Example 10: Octyl (S)-5-fluoro-3-((R)-5-isopropyl-3-(isoquinolin-1-yl)-4,5-dihydroisoxazole-5-carboxamido)-4-oxopentanoate

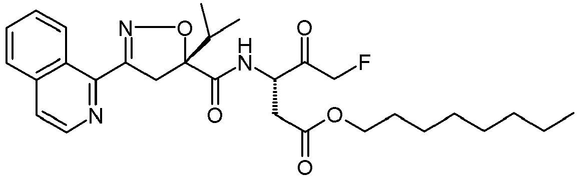

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and octanol (7.6 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.2 g (yield: 68%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.05 (d, 1H), 3.98 (m, 2H), 3.82 (d, 1H), 3.01 (dd, 2H), 2.41 (m, 1H), 1.49 (m, 2H), 1.25 (m, 10H), 1.11 (dd, 6H), 0.85 (t, 3H)

### Example 11: Decyl (S)-5-fluoro-3-((R)-5-isopropyl-3-(isoauinolin-1-yl)-4,5-dihydroisoxazole-5-carboxamido)-4-oxopentanoate

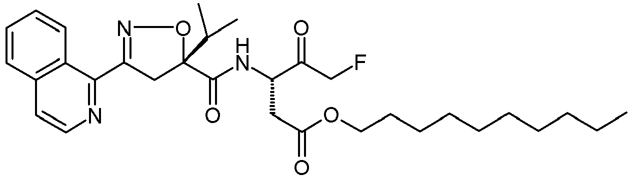

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and decanol (9.2 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 1.71 g (yield: 27%) of the title compound.

¹H NMR (400 MHz, CDCl₃) δ 9.14 (d, 1H), 8.55 (d, 1H), 7.88 (d, 1H), 7.75-7.66 (m, 4H), 5.20 (m, 2H), 4.95 (m, 1H), 4.04 (d, 1H), 3.98 (m, 2H), 3.81 (d, 1H), 3.00 (dd, 2H), 2.41 (m, 1H), 1.47 (m, 2H), 1.28-1.17 (m, 14H), 1.10 (dd, 6H), 0.87 (t, 3H)

### Example 12: Dodecyl (S)-5-fluoro-3-((R)-5-isopropyl-3-(isoquinolin-1-yl)-4,5-dihydroisoxazole-5-carboxamido)-4-oxopentanoate

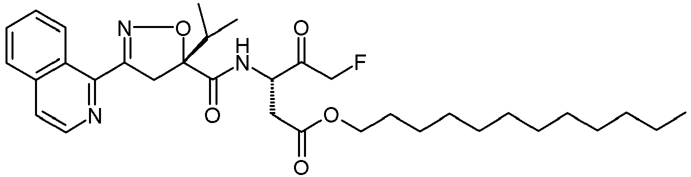

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and dodecanol (5.4 mL, 24.0 mmol, 2 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 2.4 g (yield: 34%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.05 (d, 1H), 3.97 (m, 2H), 3.82 (d, 1H), 3.00 (dd, 2H), 2.41 (m, 1H), 1.48 (m, 2H), 1.30-1.18 (m, 18H), 1.11 (dd, 6H), 0.88 (t, 3H)

### Example 13: Pentadecyl (S)-5-fluoro-3-((R)-5-isopropyl-3-(isoquinolin-1-yl)-4,5-dihydroisoxazole-5-carboxamido)-4-oxopentanoate

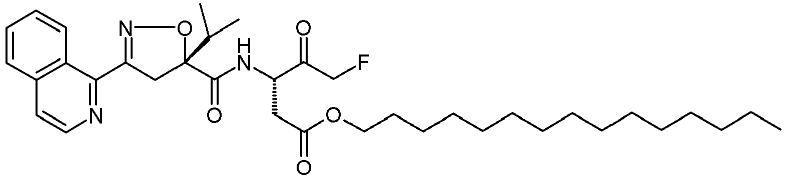

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and pentadecanol (11.0 g, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was subjected to column separation by the use of a 1:2 mixture of ethyl acetate and hexane (EtOAc: hexane=1:2) to obtain 5.6 g (yield: 75%) of the title compound.

¹H NMR (400 MHz, CDCl₃) δ 9.14 (d, 1H), 8.55 (d, 1H), 7.88 (d, 1H), 7.75-7.66 (m, 4H), 5.21 (m, 2H), 4.95 (m, 1H), 4.04 (d, 1H), 3.99 (m, 2H), 3.81 (d, 1H), 3.01 (dd, 2H), 2.41 (m, 1H), 1.48 (m, 2H), 1.30-1.13 (m, 24H), 1.11 (dd, 6H), 0.89 (t, 3H)

### Example 14: Octadecyl (S)-5-fluoro-3-((R)-5-isopropyl-3-(isoquinolin-1-yl)-4,5-dihydroisoxazole-5-carboxamido)-4-oxopentanoate

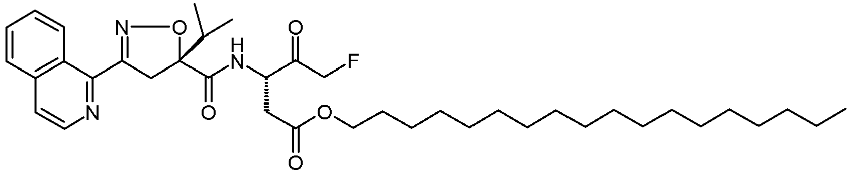

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and octadecanol (6.5 g, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was subjected to column separation by the use of a 1:2 mixture of ethyl acetate and hexane (EtOAc: hexane=1:2) to obtain 2.1 g (yield: 26%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.06 (d, 1H), 3.97 (m, 2H), 3.82 (d, 1H), 3.00 (dd, 2H), 2.41 (m, 1H), 1.48 (m, 2H), 1.30-1.18 (m, 30H), 1.10 (dd, 6H), 0.88 (t, 3H)

### Example 15: (9E,12E)-octadeca-9,12-dien-1-yl (S)-S-fluoro-3-((R)-S-isopropyl-3-(isoquinolin-1-yl)-4,5-dihydroisoxazole-5-carboxamido)-4-oxopentanoate

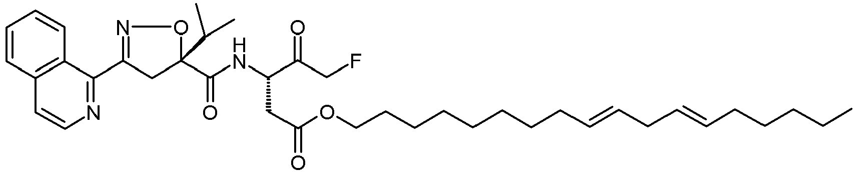

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and linoleyl alcohol (11.0 g, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was subjected to column separation by the use of a 1:2 mixture of ethyl acetate and hexane (EtOAc: hexane=1:2) to obtain 0.1 g of the title compound.

¹H NMR (400 MHz, CDCl₃) δ 9.14 (d, 1H), 8.56 (d, 1H), 7.88 (d, 1H), 7.75-7.66 (m, 4H), 5.35 (m, 4H), 5.21 (m, 2H), 4.95 (m, 1H), 4.05 (d, 1H), 3.98 (m, 2H), 3.82 (d, 1H), 3.00 (dd, 2H), 2.78 (t, 2H), 2.41 (m, 1H), 2.04 (m, 4H), 1.48 (m, 2H), 1.33-1.18 (m, 16H), 1.11 (dd, 6H), 0.88 (t, 3H)

### Example 16: Cyclopropylmethyl (S)-5-fluoro-3-((R)-5-isopropyl-3-(isoquinolin-1-yl)-4,5-dihydroisoxazole-5-carboxamido)-4-oxopentanoate

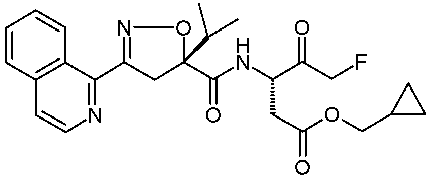

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and cyclopropylmethanol (3.9 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.3 g (yield: 76%) of the title compound.

¹H NMR (400 MHz, CDCl₃) δ 9.14 (d, 1H), 8.56 (d, 1H), 7.88 (d, 1H), 7.76-7.66 (m, 4H), 5.21 (m, 2H), 4.95 (m, 1H), 4.05 (d, 1H), 3.84 (m, 2H), 3.82 (d, 1H), 3.01 (dd, 2H), 2.41 (m, 1H), 1.10 (dd, 6H), 0.98 (m, 1H), 0.45 (d, 2H), 0.15 (d, 2H)

### Example 17: Cyclobutylmethyl (S)-5-fluoro-3-((R)-5-isopropyl-3-(isoquinolin-1-yl)-4,5-dihydroisoxazole-5-carboxamido)-4-oxopentanoate

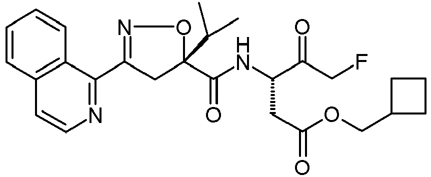

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and cyclobutylmethanol (5.1 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 3.9 g (yield: 67%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.05 (d, 1H), 3.96 (m, 2H), 3.82 (d, 1H), 3.01 (dd, 2H), 2.42 (m, 2H), 1.92 (m, 2H), 1.75 (m, 2H), 1.58 (m, 2H) 1.10 (dd, 6H)

### Example 18: Cyclopentylmethyl (S)-5-fluoro-3-((R)-5-isopropyl-3-(isoquinolin-1-yl)-4,5-dihydroisoxazole-5-carboxamido)-4-oxopentanoate

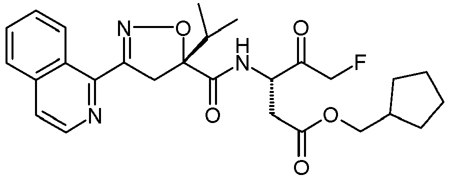

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and cyclopentylmethanol (5.2 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.2 g (yield: 69%) of the title compound.

¹H NMR (400 MHz, CDCl₃) δ 9.15 (d, 1H), 8.56 (d, 1H), 7.88 (d, 1H), 7.75-7.66 (m, 4H), 5.21 (m, 2H), 4.95 (m, 1H), 4.04 (d, 1H), 3.86 (m, 2H), 3.82 (d, 1H), 3.01 (dd, 2H), 2.42 (m, 1H), 2.03 (m, 1H), 1.59 (m, 4H), 1.43 (m, 4H) 1.10 (dd, 6H)

### Example 19: Allyl (S)-5-fluoro-3-((R)-5-isopropyl-3-(isoquinolin-1-yl)-4,5-dihydroisoxazole-5-carboxamido)-4-oxopentanoate

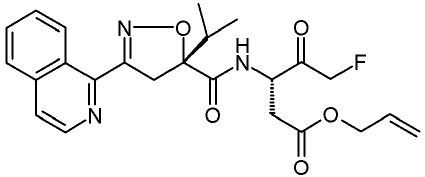

The compound of Formula 2 (5 g, 12.0 mmol) was dissolved in dichloromethane (50 mL), and then oxalyl chloride (1.6 mL, 18.0 mmol, 1.5 equiv) and dimethylformamide (0.04 mL, 0.6 mmol, 0.05 equiv) were added thereto while keeping the temperature of 5°C or lower. The reaction mixture was stirred at 25°C for about 1 hour and then distilled under reduced pressure. After dissolving in dichloromethane (50 mL), the temperature of the mixture was adjusted to 5°C, and allyl alcohol (2.0 mL, 48.0 mmol, 4 equiv) was added. After the reaction mixture was stirred at 25°C for 2 hours, 10% aqueous sodium hydrogen carbonate solution (30 mL) was added to terminate the reaction. The organic layer was separated and distilled under reduced pressure. The obtained mixture was recrystallized in a 1:4 mixture of ethanol and hexane (EtOH: hexane=1:4) to obtain 4.3 g (yield: 78%) of the title compound.

¹H NMR (400 MHz, CDCl₃) δ 9.14 (d, 1H), 8.56 (d, 1H), 7.88 (d, 1H), 7.75-7.66 (m, 4H), 5.75 (m, 1H), 5.21 (m, 4H), 4.95 (m, 1H), 4.50 (m, 2H), 4.04 (d, 1H), 3.82 (d, 1H), 3.01 (dd, 2H), 2.42 (m, 1H), 1.10 (dd, 6H)

### Example 20: Isopropyl (S)-5-fluoro-3-((R)-5-isopropyl-3-(isoquinolin-1-yl)-4,5-dihydroisoxazole-5-carboxamido)-4-oxopentanoate

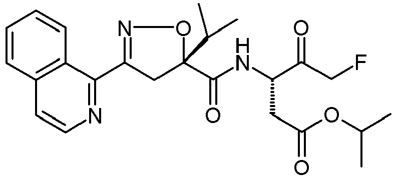

The compound of Formula 2 (0.5 g, 1.2 mmol) was dissolved in dimethylformamide (5 mL), and then isopropyl promide (0.18 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 3.6 g (yield: 27%) of the title compound.

¹H NMR (400 MHz, CDCl₃) δ 9.14 (d, 1H), 8.56 (d, 1H), 7.88 (d, 1H), 7.75-7.66 (m, 4H), 5.15 (m, 3H), 4.95 (m, 1H), 4.03 (d, 1H), 3.82 (d, 1H), 3.01 (dd, 2H), 2.40 (m, 1H), 1.23 (m, 6H) 1.11 (dd, 6H)

### Example 21: Penta-3-yl (S)-5-fluoro-3-((R)-5-isopropyl-3-(isoquinolin-1-yl)-4,5 dihydroisoxazole-5-carboxamido)-4-oxopentanoate

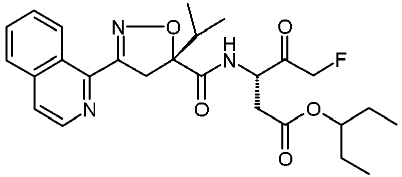

The compound of Formula 2 (0.5 g, 1.2 mmol) was dissolved in dimethylformamide (5 mL), and then 3-bromopentane (0.22 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.05 g (yield: 9%) of the title compound.

¹H NMR (400 MHz, CDCl₃) δ 9.14 (d, 1H), 8.56 (d, 1H), 7.88 (d, 1H), 7.75-7.66 (m, 4H), 5.20 (m, 2H), 4.95 (m, 1H), 4.76 (m, 1H), 4.03 (d, 1H), 3.82 (d, 1H), 3.01 (dd, 2H), 2.40 (m, 1H), 1.54 (m, 4H), 1.10 (dd, 6H), 0.86 (t, 6H)

### Example 22: Sec-butyl (S)-5-fluoro-3-((R)-5-isopropyl-3-(isoquinolin-1-yl)-4,5-dihydroisoxazole-5-carboxamido)-4-oxopentanoate

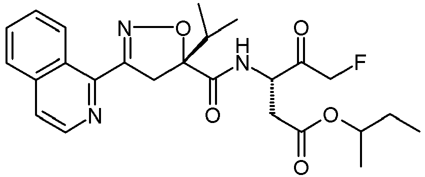

The compound of Formula 2 (0.5 g, 1.2 mmol) was dissolved in dimethylformamide (5 mL), and then 2-bromobutane (0.18 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.5 g (yield: 88%) of the title compound.

¹H NMR (400 MHz, CDCl₃) δ 9.15 (d, 1H), 8.56 (d, 1H), 7.88 (d, 1H), 7.75-7.66 (m, 4H), 5.20 (m, 2H), 4.96 (m, 1H), 4.86 (m, 1H), 4.03 (d, 1H), 3.82 (d, 1H), 3.01 (dd, 2H), 2.40 (m, 1H), 1.54 (m, 2H), 1.21 (m, 3H), 1.10 (dd, 6H), 0.89 (t, 3H)

### Example 23: Pentan-2-yl (S)-5-fluoro-3-((R)-5-isopropyl-3-(isoquinolin-1-yl)-4,5-dihydroisoxazole-5-carboxamido)-4-oxopentanoate

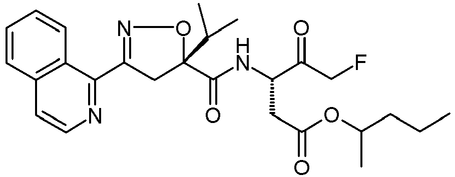

The compound of Formula 2 (0.5 g, 1.2 mmol) was dissolved in dimethylformamide (5 mL), and then 1-bromopentane (0.19 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.14 g (yield: 24%) of the title compound.

¹H NMR (400 MHz, CDCl₃) δ 9.15 (d, 1H), 8.56 (d, 1H), 7.88 (d, 1H), 7.77-7.66 (m, 4H), 5.20 (m, 2H), 4.96 (m, 1H), 4.86 (m, 1H), 4.04 (d, 1H), 3.82 (d, 1H), 3.01 (dd, 2H), 2.40 (m, 1H), 1.51 (m, 2H), 1.40 (m, 2H), 1.23 (m, 3H), 1.10 (dd, 6H), 0.89 (t, 3H)

### Example 24: Heptan-2-yl (S)-5-fluoro-3-((R)-5-isopropyl-3-(isoquinolin-1-yl)-4,5-dihydroisoxazole-5-carboxamido)-4-oxopentanoate

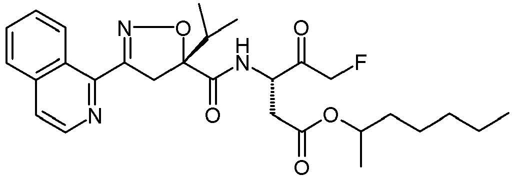

The compound of Formula 2 (0.5 g, 1.2 mmol) was dissolved in dimethylformamide (5 mL), and then 3-bromohepntane (0.18 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.12 g (yield: 20%) of the title compound.

¹H NMR (400 MHz, CDCl₃) δ 9.15 (d, 1H), 8.56 (d, 1H), 7.88 (d, 1H), 7.77-7.66 (m, 4H), 5.21 (m, 2H), 4.96 (m, 1H), 4.84 (m, 1H), 4.05 (d, 1H), 3.82 (d, 1H), 3.01 (dd, 2H), 2.41 (m, 1H), 1.49 (m, 2H), 1.30 (m, 4H), 1.22 (m, 3H), 1.11 (dd, 6H), 0.89 (t, 3H)

### Example 25: Cyclopentyl (S)-5-fluoro-3-((R)-5-isopropyl-3-(isoauinolin-1-yl)-4,5-dihydroisoxazole-5-carboxamido)-4-oxopentanoate

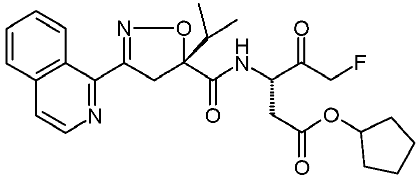

The compound of Formula 2 (0.5 g, 1.2 mmol) was dissolved in dimethylformamide (5 mL), and then 3-cyclopentyl bromide (0.19 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.20 g (yield: 35%) of the title compound.

¹H NMR (400 MHz, CDCl₃) δ 9.15 (d, 1H), 8.56 (d, 1H), 7.88 (d, 1H), 7.77-7.66 (m, 4H), 5.21 (m, 2H), 4.96 (m, 1H), 4.84 (m, 1H), 4.05 (d, 1H), 3.84 (d, 1H), 3.06 (dd, 2H), 2.41 (m, 1H), 1.72-1.23 (m, 8H), 1.11 (dd, 6H)

### Example 26: Cyclohexyl (S)-5-fluoro-3-((R)-5-isopropyl-3-(isoquinolin-1-yl)-4,5-dihydroisoxazole-5-carboxamido)-4-oxopentanoate

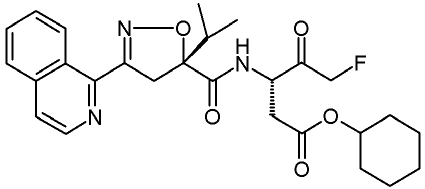

The compound of Formula 2 (0.5 g, 1.2 mmol) was dissolved in dimethylformamide (5 mL), and then 3-cyclohexyl iodide (0.23 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.06 g (yield: 10%) of the title compound.

¹H NMR (400 MHz, CDCl₃) δ 9.15 (d, 1H), 8.56 (d, 1H), 7.88 (d, 1H), 7.77-7.66 (m, 4H), 5.21 (m, 2H), 4.96 (m, 1H), 4.84 (m, 1H), 4.05 (d, 1H), 3.83 (d, 1H), 3.07 (dd, 2H), 2.41 (m, 1H), 1.73-1.22 (m, 10H), 1.11 (dd, 6H)

### Example 27: Benzyl (S)-5-fluoro-3-((R)-5-isopropyl-3-(isoquinolin-1-yl)-4,5-dihydroisoxazole-5-carboxamido)-4-oxopentanoate

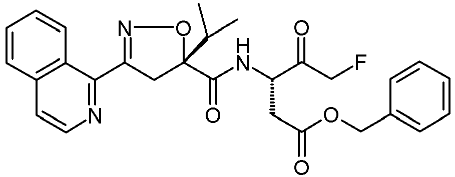

The compound of Formula 2 (0.5 g, 1.2 mmol) was dissolved in dimethylformamide (5 mL), and then benzyl bromide (0.18 mL, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.5 g (yield: 78%) of the title compound.

¹H NMR (400 MHz, CDCl₃) δ 9.14 (d, 1H), 8.56 (d, 1H), 7.88 (d, 1H), 7.75-7.66 (m, 4H), 7.36 (m, 3H), 7.22 (m, 2H), 5.17 (m, 2H), 5.05 (m, 2H), 4.95 (m, 1H), 4.05 (d, 1H), 3.82 (d, 1H), 3.01 (dd, 2H), 2.40 (m, 1H), 1.11 (dd, 6H)

### Example 28: (5-methyl-2-oxo-1,3-dioxol-4-yl)methyl (S)-S-fluoro-3-((R)-S-isopropyl-3-(isoquinolin-1-yl)-4,5-dihydroisoxazole-5-carboxamido)-4-oxopentanoate

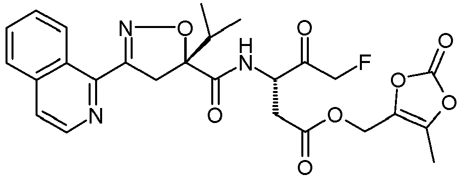

The compound of Formula 2 (0.5 g, 1.2 mmol) was dissolved in dimethylformamide (5 mL), and then 4-chloromethyl-5-methyl-1,3-dioxol-2-one (0.19 g, 1.8 mmol, 1.5 equiv) and potassium carbonate (0.2 g, 1.8 mmol, 1.2 equiv) was added thereto. The reaction mixture was stirred at 25°C for about 18 hours, diluted in ethyl acetate (EtOAc, 30 mL), and 10% aqueous sodium hydrogen carbonate solution (30 mL) was added and reacted with stirring. After adding water (30 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.21 g (yield: 33%) of the title compound.

¹H NMR (400 MHz, CDCl₃) δ 9.14 (d, 1H), 8.57 (d, 1H), 7.89 (d, 1H), 7.75-7.66 (m, 4H), 5.17 (m, 2H), 4.87 (m, 1H), 4.42 (s, 2H), 4.06 (d, 1H), 3.00 (dd, 2H), 2.39 (m, 1H), 2.18 (s, 3H), 1.11 (dd, 6H)

### Example 29: 2-Methoxyphenyl (S)-5-fluoro-3-((R)-5-isopropyl-3-(isoquinolin-1-yl)-4,5-dihydroisoxazole-5-carboxamido)-4-oxopentanoate

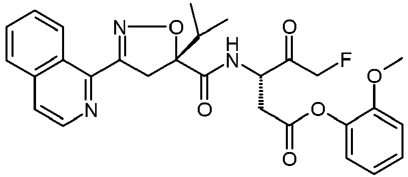

The compound of Formula 2 (10 g, 24.0 mmol) was dissolved in dichloromethane (100 mL), and then 2-methoxyphenol (11.9 g, 96.0 mmol, 4 equiv), hydroxybenzotriazole (HOBt, 0.64 g, 0.48 mmol, 0.2 equiv) and triethylamine (0.6 mL, 0.48 mmol, 0.2 equiv) were added, and N-ethyl-N'-(3-dimethylaminopropyl)carbodiimide hydrochloride (EDCI, 5.5 g, 28.8 mmol, 1.2 eq) was added thereto while keeping the temperature of 5°C or lower. The reaction mixture was stirred at 25°C for about 6 hours, and 10% aqueous sodium hydrogen carbonate solution (50 mL) was added to terminate the reaction. After adding water (50 mL) and stirring, the organic layer was separated and distilled under reduced pressure. The obtained mixture was subjected to column separation by the use of a 1:2 mixture of ethyl acetate and hexane (EtOAc: hexane=1:2) to obtain 3.6 g (yield: 27%) of the title compound.

¹H NMR (400 MHz, CDCl₃) δ 9.15 (d, 1H), 8.57 (d, 1H), 7.88 (d, 1H), 7.77-7.66 (m, 4H), 7.19 (m, 2H), 6.94 (m, 2H), 5.16 (m, 1H), 4.58 (m, 2H), 4.10 (d, 1H), 3.91 (s, 3H), 3.84 (d, 1H), 3.01 (dd, 2H), 2.44 (m, 1H), 1.11 (dd, 6H)

### Example 30: 2,3-dihydro-1H-inden-5-yl (S)-5-ftuoro-3-((R)-5-isopropyl-3-(isoquinolin-1-yl)-4,5-dihydroisoxazole-5-carboxamido)-4-oxopentanoate

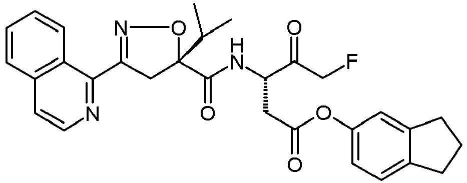

The compound of Formula 2 (10 g, 24.0 mmol) was dissolved in dichloromethane (100 mL), and then 5-indazole (12.8 g, 96.0 mmol, 4 equiv), hydroxybenzotriazole (0.64 g, 0.48 mmol, 0.2 equiv) and triethylamine (0.6 mL, 0.48 mmol, 0.2 equiv) were added, and EDCI (5.5 g, 28.8 mmol, 1.2 eq) was added thereto while keeping the temperature of 5°C or lower. The reaction mixture was stirred at 25°C for about 6 hours, and 10% aqueous sodium hydrogen carbonate solution (50 mL) was added to terminate the reaction. After adding water (50 mL) and stirring, the organic layer was separated and distilled under reduced pressure. The obtained mixture was subjected to column separation by the use of a 1:2 mixture of ethyl acetate and hexane (EtOAc: hexane=1:2) to obtain 3.4 g (yield: 28%) of the title compound.

¹H NMR (400 MHz, CDCl₃) δ 9.15 (d, 1H), 8.57 (d, 1H), 7.88 (d, 1H), 7.77-7.66 (m, 4H), 7.18 (d, 1H), 7.03 (s, 1H), 6.93 (d, 1H), 5.17 (m, 1H), 4.63 (m, 2H), 4.15 (d, 1H), 3.85 (d, 1H), 3.04 (dd, 2H), 2.90 (m, 4H), 2.45 (m, 1H), 2.13 (m, 2H), 1.11 (dd, 6H)

### Example 31: Naphthalen-1-yl (S)-5-fluoro-3-((R)-5-isopropyl-3-(isoquinolin-1-yl)-4,5-dihydroisoxazole-5-carboxamido)-4-oxopentanoate

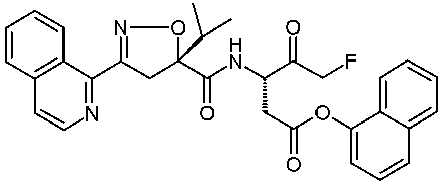

The compound of Formula 2 (0.5 g, 1.2 mmol) was dissolved in dichloromethane (10 mL), and then 1-naphthol (0.69 g, 4.8 mmol, 4 equiv), hydroxybenzotriazole (0.64 g, 0.24 mmol, 0.2 equiv) and triethylamine (0.6 mL, 0.24 mmol, 0.2 equiv) were added, and EDCI (0.28 g, 1.4 mmol, 1.2 eq) was added thereto while keeping the temperature of 5°C or lower. The reaction mixture was stirred at 25°C for about 6 hours, and 10% aqueous sodium hydrogen carbonate solution (10 mL) was added to terminate the reaction. After adding water (10 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.07 g (yield: 10%) of the title compound.

¹H NMR (400 MHz, CDCl₃) δ 9.15 (d, 1H), 8.57 (d, 1H), 8.25 (d, 1H), 7.95 (d, 1H), 7.88 (d, 1H), 7.74-7.57 (m, 7H), 7.47 (m, 2H), 5.32 (m, 1H), 4.66 (m, 2H), 4.13 (d, 1H), 3.88 (d, 1H), 3.10 (dd, 2H), 2.54 (m, 1H), 1.11 (dd, 6H)

### Example 32: Phenyl (S)-5-fluoro-3-((R)-5-isopropyl-3-(isoquinolin-1-yl)-4,5-dihydroisoxazole-5-carboxamido)-4-oxopentanoate

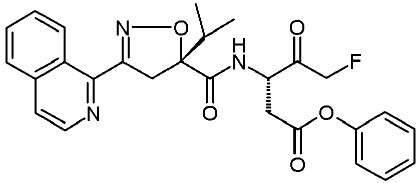

The compound of Formula 2 (10 g, 24.0 mmol) was dissolved in dichloromethane (100 mL), and then phenol (9.0 g, 96.0 mmol, 4 equiv), hydroxybenzotriazole (0.64 g, 0.48 mmol, 0.2 equiv) and triethylamine (0.6 mL, 0.48 mmol, 0.2 equiv) were added, and EDCI (5.5 g, 28.8 mmol, 1.2 eq) was added thereto while keeping the temperature of 5°C or lower. The reaction mixture was stirred at 25°C for about 6 hours, and 10% aqueous sodium hydrogen carbonate solution (50 mL) was added to terminate the reaction. After adding water (50 mL) and stirring, the organic layer was separated and distilled under reduced pressure. The obtained mixture was recrystallized in a 1:5 mixture of ethanol and hexane (EtOH: hexane=1:5) to obtain 2.9 g (yield: 25%) of the title compound.

¹H NMR (400 MHz, CDCl₃) δ 9.15 (d, 1H), 8.57 (d, 1H), 7.88 (d, 1H), 7.77-7.66 (m, 4H), 7.38 (m, 2H), 7.20 (m, 3H), 5.19 (m, 1H), 4.62 (m, 2H), 4.11 (d, 1H), 3.82 (d, 1H), 3.03 (dd, 2H), 2.45 (m, 1H), 1.11 (dd, 6H)

### Example 33: Naphthalen-1-ylmethyl (S)-5-fluoro-3-((R)-5-isopropyl-3-(isoquinolin-1-yl)-4,5-dihydroisoxazole-5-carboxamido)-4-oxopentanoate

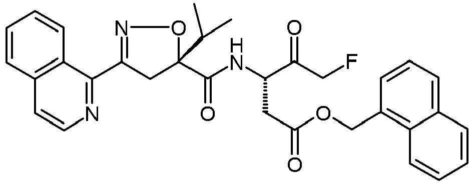

The compound of Formula 2 (0.5 g, 1.2 mmol) was dissolved in dichloromethane (10 mL), and then 1-naphthalenemethanol (0.38 g, 4.8 mmol, 4 equiv), 4-dimethylaminopyridine (DMAP, 0.03 g, 0.24 mmol, 0.2 equiv) and triethylamine (0.6 mL, 0.24 mmol, 0.2 equiv) were added, and EDCI (0.28 g, 1.4 mmol, 1.2 eq) was added thereto while keeping the temperature of 5°C or lower. The reaction mixture was stirred at 25°C for about 6 hours, and 10% aqueous sodium hydrogen carbonate solution (10 mL) was added to terminate the reaction. After adding water (10 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.2 g (yield: 33%) of the title compound.

¹H NMR (400 MHz, CDCl₃) δ 9.16 (d, 1H), 8.57 (d, 1H), 7.88 (d, 1H), 7.81-7.55 (m, 7H), 7.52 (m, 2H), 7.43 (m, 2H), 5.58 (m, 2H), 5.17 (m, 1H), 5.02 (m, 2H), 4.08 (d, 1H), 3.85 (d, 1H), 3.03 (dd, 2H), 2.43 (m, 1H), 1.11 (dd, 6H)

### Example 34: 2,2,2-trifluoroethyl (S)-5-fluoro-3-((R)-5-isopropyl-3-(isoquinolin-1-yl)-4,5-dihydroisoxazole-5-carboxamido)-4-oxopentanoate

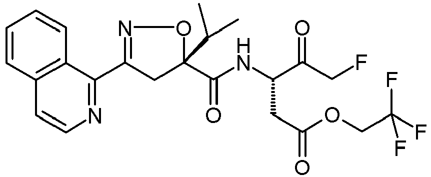

The compound of Formula 2 (0.5 g, 1.2 mmol) was dissolved in dichloromethane (10 mL), and then trifluoroethanol (0.35 mL, 4.8 mmol, 4 equiv), hydroxybenzotriazole (0.64 g, 0.24 mmol, 0.2 equiv) and triethylamine (0.6 mL, 0.24 mmol, 0.2 equiv) were added, and EDCI (0.28 g, 1.4 mmol, 1.2 eq) was added thereto while keeping the temperature of 5°C or lower. The reaction mixture was stirred at 25°C for about 6 hours, and 10% aqueous sodium hydrogen carbonate solution (10 mL) was added to terminate the reaction. After adding water (10 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.1 g (yield: 17%) of the title compound.

¹H NMR (400 MHz, CDCl₃) δ 9.14 (d, 1H), 8.56 (d, 1H), 7.88 (d, 1H), 7.76-7.65 (m, 4H), 4.88 (m, 1H), 4.70 (m, 2H), 4.12 (m, 2H), 4.06 (d, 1H), 3.82 (d, 1H), 3.06 (dd, 2H), 2.40 (m, 1H), 1.11 (dd, 6H)

### Example 35: 2-Methoxyethyl (S)-5-fluoro-3-((R)-5-isopropyl-3-(isoquinolin-1-yl)-4,5-dihydroisoxazole-5-carboxamido)-4-oxopentanoate

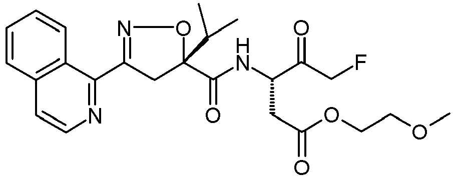

The compound of Formula 2 (0.5 g, 1.2 mmol) was dissolved in dichloromethane (10 mL), and then methoxyethanol (0.37 mL, 4.8 mmol, 4 equiv), hydroxybenzotriazole (0.64 g, 0.24 mmol, 0.2 equiv) and triethylamine (0.6 mL, 0.24 mmol, 0.2 equiv) were added, and EDCI (0.28 g, 1.4 mmol, 1.2 eq) was added thereto while keeping the temperature of 5°C or lower. The reaction mixture was stirred at 25°C for about 6 hours, and 10% aqueous sodium hydrogen carbonate solution (10 mL) was added to terminate the reaction. After adding water (10 mL) and stirring, the organic layer was separated and distilled under reduced pressure. The obtained mixture was subjected to column separation by the use of a 1:2 mixture of ethyl acetate and hexane (EtOAc: hexane=1:2) to obtain 0.14 g (yield: 25%) of the title compound.

¹H NMR (400 MHz, CDCl₃) δ 9.15 (d, 1H), 8.56 (d, 1H), 7.88 (d, 1H), 7.75-7.65 (m, 4H), 5.21 (m, 2H), 4.92 (m, 1H), 4.17 (m, 2H), 4.05 (d, 1H), 3.82 (d, 1H), 3.47 (m, 2H), 3.30 (S, 3H), 3.05 (dd, 2H), 2.42 (m, 1H), 1.11 (dd, 6H)

### Example 36: 2-Fluoroethyl (S)-5-fluoro-3-((R)-5-isopropyl-3-(isoquinolin-1-yl)-4,5-dihydroisoxazole-5-carboxamido)-4-oxopentanoate

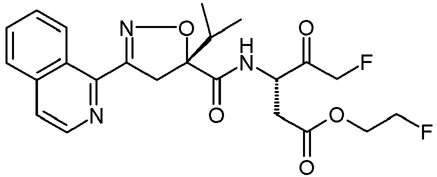

The compound of Formula 2 (100 mg, 0.24 mmol) was reacted with EDCI (69 mg, 0.36 mmol), DMAP (3 mg, 0.02 mmol) and 2-fluoroethanol (0.14 mL, 2.41 mmol) in dichloromethane (4 mL) under room temperature condition for 2 hours. After adding water, the reaction mixture was extracted with EtOAc. The organic layer was dried over sodium sulfate, concentrated and purified by the use of medium pressure liquid chromatography (MPLC) to obtain the title compound (76 mg, 68%).

¹H NMR (CDCl₃) δ 9.12 (t, 1H), 8.55 (d, 1H), 7.86 ∼ 7.66 (m, 5H), 5.28 ∼ 4.88 (m, 3H), 4.64 ∼ 4.30 (m, 4H), 4.01 (dd, 1H), 3.79 (dd, 1H), 3.16 ∼ 2.94(m, 2 H), 2.41(p, 1H), 1.08 ∼ 1.07 (m, 6H)

### Example 37: Neopentyl (S)-5-fluoro-3-((R)-5-isopropyl-3-(isoquinolin-1-yl)-4,5-dihydroisoxazole-5-carboxamido)-4-oxopentanoate

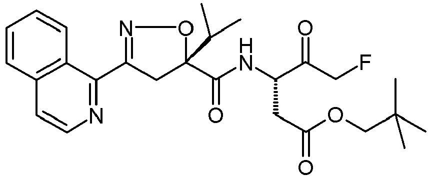

The compound of Formula 2 (2.0 g, 4.81 mmol) was reacted with EDCI (1.4 g, 7.22 mmol), DMAP (118 mg, 0.96 mmol) and 2,2-dimethylpropan-1-ol (5.19 mL, 48.1 mmol) in dichloromethane (80 mL) under room temperature condition for 18 hours. After adding water, the reaction mixture was extracted with EtOAc. The organic layer was dried over sodium sulfate, concentrated and purified by the use of MPLC to obtain the title compound (0.94 g, 40%).

¹H NMR (CDCl₃) δ 9.13 (t, 1H), 8.54 (d, 1H), 7.86 ∼ 7.66 (m, 5H), 5.28 ∼ 4.91 (m, 3H), 4.01 (dd, 1H), 3.82 ∼ 3.79 (m, 2H), 3.67 (q, 1H), 3.11 ∼ 2.90 (m, 2H), 2.37(p, 1H), 1.10 ∼ 1.04 (m, 6H), 0.91(s, 5H), 0.79(s, 4H)

### Example 38: Thiophen-2-ylmethyl (S)-5-fluoro-3-((R)-5-isopropyl-3-(isoquinolin-1-yl)-4,5-dihydroisoxazole-5-carboxamido)-4-oxopentanoate

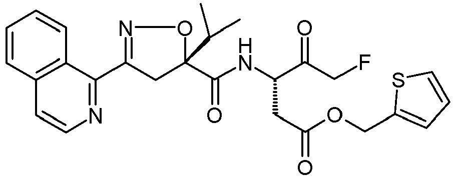

The compound of Formula 2 (500 mg, 1.20 mmol) was reacted with EDCI (277 mg, 1.44 mmol), DMAP (37 mg, 0.24 mmol) and thiophen-2-ylmethanol (550 mg, 4.81 mmol) in dichloromethane (4 mL) under room temperature condition for 12 hours. After adding water, the reaction mixture was extracted with EtOAc. The organic layer was dried over sodium sulfate, concentrated and purified by the use of MPLC to obtain the title compound (306 mg, 50%).

¹H NMR (CDCl₃) δ 9.15 (t, 1H), 8.56 (d, 1H), 7.88 ∼ 7.67 (m, 4H), 7.38 ∼ 6.99 (m, 4H), 5.32 ∼ 4.90 (m, 5H), 4.01 (dd, 1H), 3.80 (dd, 1H), 3.13 ∼ 2.88(m, 2H), 2.42 ∼ 2.32(m, 1H), 1.10 ∼ 1.03 (m, 6H)

### Example 39: Thiophen-3-ylmethyl (S)-5-fluoro-3-((R)-5-isopropyl-3-(isoquinolin-1-yl)-4,5-dihydroisoxazole-5-carboxamido)-4-oxopentanoate

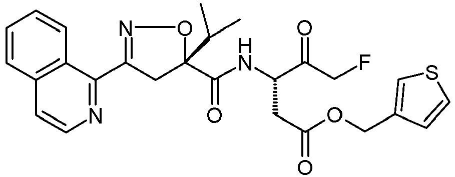

The compound of Formula 2 (500 mg, 1.20 mmol) was reacted with EDCI (277 mg, 1.44 mmol), DMAP (37 mg, 0.24 mmol) and thiophen-3-ylmethanol (550 mg, 4.81 mmol) in dichloromethane (4 mL) under room temperature condition for 12 hours. After adding water, the reaction mixture was extracted with EtOAc. The organic layer was dried over sodium sulfate, concentrated and purified by the use of MPLC to obtain the title compound (243 mg, 40%).

¹H NMR (CDCl₃) δ 9.14 (t, 1H), 8.54 (d, 1H), 7.87 ∼ 7.64 (m, 4H), 7.37 ∼ 7.17 (m, 4H), 5.15 ∼ 4.90 (m, 4H), 4.01 (dd, 1H), 3.78 (dd, 1H), 3.14 ∼ 2.87(m, 2H), 2.40 ∼ 2.33(m, 1H), 1.09 ∼ 1.02 (m, 6H)

### Example 40: Furan-3-ylmethyl (S)-5-fluoro-3-((R)-5-isopropyl-3-(isoquinolin-1-yl)-4,5-dihydroisoxazole-5-carboxamido)-4-oxopentanoate

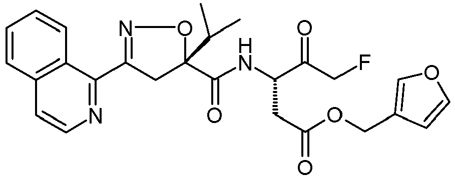

The compound of Formula 2 (500 mg, 1.20 mmol) was reacted with EDCI (277 mg, 1.44 mmol), DMAP (37 mg, 0.24 mmol) and furan-3-ylmethanol (471 mg, 4.81 mmol) in dichloromethane (4 mL) under room temperature condition for 12 hours. After adding water, the reaction mixture was extracted with EtOAc. The organic layer was dried over sodium sulfate, concentrated and purified by the use of MPLC to obtain the title compound (269 mg, 44%).

¹H-NMR (CDCl₃) δ 9.13 (t, 1H), 8.54 (d, 1H), 7.87 ∼ 7.67 (m, 4H), 7.40 ∼ 7.35 (m, 3H), 6.54 (m, 1H), 5.32 ∼ 4.56 (m, 5H), 4.01 (dd, 1H), 3.77 (dd, 1H), 3.11 ∼ 2.88(m, 2H), 2.40 ∼ 2.33(m, 1H), 1.10 ∼ 1.02 (m, 6H)

### Experimental Example 1: Hydrolysis test of prodrug compound of Formula 1 in plasma

Whole blood of 7-week-old male SD-rat was collected and centrifuged to obtain fresh plasma. The compound of Example 2 was selected as a test prodrug, and a 5 mg/mL DMSO stock thereof was used as a working solution. This solution was diluted 1/10 in acetonitrile to a concentration of 0.5 mg/mL, and then spiked with the fresh plasma obtained above at a ratio of 1/100 to make a final concentration of 1 µg/mL in plasma. It was set as the starting concentration for measuring drug stability in plasma. After that, 50 µl of plasma was collected at 10 sec, 5 min, 10 min, 20 min and 30 min, respectively, deproteinized with acetonitrile containing an internal standard (IS) and centrifuged, and the supernatant was analyzed by injecting into LC-MS/MS. The obtained peak area of the prodrug was corrected with the peak area of IS to obtain the peak response at each sample collection time, and the residual ratio (% remaining) compared to the initial value was converted.

In addition, by correcting the obtained peak area of the compound of Formula 2 with the peak area of IS, the peak response at each sample collection time was obtained, and the generation and disappearance patterns according to time were confirmed.

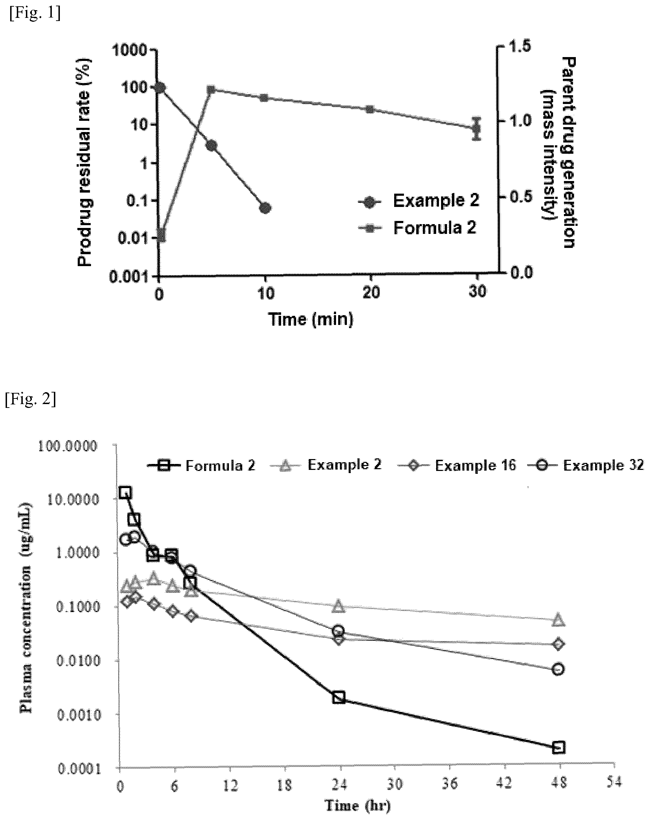

The analysis results are represented in Figure 1. According to Figure 1, when the prodrugs of Formula 1 was mixed with fresh plasma of a rat, most of them were lost within about 5 minutes, and such result was analyzed that this is because the prodrug of Formula 1 is hydrolyzed by an esterase present in plasma and converted into the compound of Formula 2.

### Experimental Example 2: Pharmacokinetic test in mice

For subcutaneous injection (SC) PK testing of the prodrug compound of Formula 1 (the compounds of Examples 2, 16 and 32), about 7-week-old C57BL6 mice were prepared, and a group separation was carried out by allocating 3 animals per administration compound. Because it was a case of subcutaneous injection, fasting was not performed. On the day of administration, a drug solution was prepared at a concentration of 5 mg/mL by the use of 0.5% methyl cellulose (MC) as a vehicle, and this was injected subcutaneously in an amount of 10 mL per kg of body weight of each individual to make a final dose of 50 mg/kg. At 1, 2, 4, 6, 8, 24 and 48 hours after administration, blood was collected through the orbital vein, and the blood of each of the three animals per group was pooled in a heparin tube. The obtained blood sample was centrifuged at 15,000 rpm for 2 minutes to separate plasma, and 50 µl was taken and stored frozen at -20°C. On the day of the analysis, the sample was thawed at room temperature, and deproteinization was carried out with 200 µl of acetonitrile which was a total of 4 times the stored volume. At this time, acetonitrile contained an internal standard and 5% formic acid (FA). To prepare a calibration curve, acetonitrile solutions (including IS and 5% FA) with known concentrations of 0.1, 0.5, 5, 50 and 500 ng/mL, respectively, were prepared, and the blank serum was deproteinized with 4 times the volume of acetonitrile as above to prepare a calibration curve of final 0.4 - 2,000 ng/mL. After injecting 0.5 µl of the supernatant obtained after deproteinization to LC-MS/MS, the peak area of the compound of Formula 2 was corrected to the IS peak area to obtain the peak response at each sample collection point, and the concentration was converted through a calibration curve. Pharmacokinetic parameters (Cₘₐₓ, Tₘₐₓ, AUCₗₐₛₜ, t_{1/2}, etc.) were calculated through a noncompartmental analysis method using WinNonlin 8.1 for the value of blood concentration according to time for each administration group. The pharmacokinetic characteristics of each compound were compared by comparing exposure and half-life changes by the drug administration group.

In the case of subcutaneous injection of a prodrug of the compound of Formula 2-which is the parent drug, the peak blood drug concentration value of the compound of Formula 2 was decreased. In addition, a tendency to increase the elimination half-life (t_{1/2}) of the drug depending on the substance was confirmed. The characteristics observed in the case of administering the prodrug are advantageous in terms of the expression of side effects caused by the high blood concentration, and it is expected to have advantages over direct administration of the parent drug to maintain the effective concentration required for the expression of efficacy and achieve the desired efficacy.

The pharmacokinetic parameters of the parent drug-which is a metabolite measured in the plasma of mice after subcutaneous administration of the compounds of Examples 2, 16 and 32, which are the prodrug compounds of Formula 1-are represented in Table 1 below.

**[Table 1]**

| | **Formula 2 Mean** | **Example 2 Mean** | **Example 16 Mean** | **Example 32 Mean** |
|---|---|---|---|---|
| **Cₘₐₓ (µg/mL)** | 12.50 | 0.33 | 1.86 | 0.15 |
| **Tₘₐₓ (hr)** | 1 | 4 | 2 | 2 |
| **AUCₗₐₛₜ (hr × µg/mL)** | 24.02 | 6.07 | 12.49 | 1.97 |
| **t_{1/2} (hr)** | 7.38 | 21.05 | 9.53 | 27.05 |

Figure 2 represents the mean plasma concentration profile over time of the compound of Formula 2 obtained after subcutaneous injection of the prodrugs of the compounds of Examples 2, 16 and 32 into C57B0L/6 mice.

### Example 41: Preparation of sustained-release microspheres for intra-articular administration using caspase inhibitor prodrugs

According to the compositions denoted in Tables 2 and 3 below, 16 types of sustained-release test microspheres for intra-articular administration encapsulated with caspase inhibitor prodrugs were prepared. First, the prodrug and PLGA (L/G ratio = 50:50, M.W. 38,000 - 54,000) were weighed in a weight ratio of 1:5, an organic solvent dichloromethane was added, and stirred to prepare the disperse phase. For the continuous phase, 150 mL of 2% polyvinyl alcohol (M.W. 31,000 - 50,000, hydrolysis degree 87 - 89%) was used, and emulsions were prepared by membrane emulsification. The prepared emulsions were stirred overnight at room temperature to remove solvent (solvent evaporation), washed with sterile purified water, and then lyophilized to obtain microspheres.

**[Table 2]**

| **Test microsphere No.** | **1** | **2** | **3** | **4** | **5** | **6** | **7** | **8** |
|---|---|---|---|---|---|---|---|---|
| **Caspase inhibitor prodrug** | Example 1 | Example 2 | Example 3 | Example 4 | Example 7 | Example 8 | Example 9 | Example 10 |
| **Prodrug amount (g)** | 0.4 | 0.4 | 0.4 | 0.4 | 0.4 | 0.4 | 0.4 | 0.4 |
| **PLGA amount (g)** | 2.0 | 2.0 | 2.0 | 2.0 | 2.0 | 2.0 | 2.0 | 2.0 |
| **PLGAL/G ratio** | 50:50 | 50:50 | 50:50 | 50:50 | 50:50 | 50:50 | 50:50 | 50:50 |
| **PLGA M.W. (kDa)** | 38-54 | 38-54 | 38-54 | 38-54 | 38-54 | 38-54 | 38-54 | 38-54 |
| **Solvent amount (g)** | 20.0 | 20.0 | 20.0 | 20.0 | 20.0 | 20.0 | 20.0 | 20.0 |
| **PVA concentration (%, w/v)** | 2 | 2 | 2 | 2 | 2 | 2 | 2 | 2 |
| **PVA solution amout (mL)** | 150 | 150 | 150 | 150 | 150 | 150 | 150 | 150 |

**[Table 3]**

| **Test microsphere No.** | **9** | **10** | **11** | **12** | **13** | **14** | **15** | **16** |
|---|---|---|---|---|---|---|---|---|
| **Caspase inhibitor prodrug** | Example 11 | Example 12 | Example 6 | Example 5 | Example 16 | Example 17 | Example 18 | Example 32 |
| **Prodrug amount (g)** | 0.4 | 0.4 | 0.4 | 0.4 | 0.4 | 0.4 | 0.4 | 0.4 |
| **PLGA amount (g)** | 2.0 | 2.0 | 2.0 | 2.0 | 2.0 | 2.0 | 2.0 | 2.0 |
| **PLGA L/G ratio** | 50:50 | 50:50 | 50:50 | 50:50 | 50:50 | 50:50 | 50:50 | 50:50 |
| **PLGA M.W. (kDa)** | 38-54 | 38-54 | 38-54 | 38-54 | 38-54 | 38-54 | 38-54 | 38-54 |
| **Solvent amount (g)** | 20.0 | 20.0 | 20.0 | 20.0 | 20.0 | 20.0 | 20.0 | 20.0 |
| **PVA concentration (%, w/v)** | 2 | 2 | 2 | 2 | 2 | 2 | 2 | 2 |
| **PVA solution amout (mL)** | 150 | 150 | 150 | 150 | 150 | 150 | 150 | 150 |

### Example 42: Preparation of sustained-release microspheres for intra-articular administration using caspase inhibitor

According to the composition denoted in Table 4 below, 2 types of sustained-release comparative microspheres for intra-articular administration encapsulated with caspase inhibitor nivocasan were prepared. The dispersed phase was prepared by weighing the caspase inhibitor and PLGA in a weight ratio of 1:5, adding dichloromethane as an organic solvent, and stirring. At this time, the L/G ratio of the PLGA used was two types of 50:50 (M.W. 38,000 - 54,000) and 75:25 (M.W. 76,000 - 115,000). For the continuous phase, 150 mL of 2% polyvinyl alcohol (M.W. 31,000 - 50,000, hydrolysis degree 87 - 89 %) was used, and an emulsion was prepared by a membrane emulsification method. The prepared emulsion was stirred overnight at room temperature to remove the solvent, washed with sterile purified water repeatedly, and then freeze-dried to prepare microspheres.

**[Table 4]**

| **Comparative microsphere No.** | **1** | **2** |
|---|---|---|
| **Nivocasan amount (g)** | 0.4 | 0.4 |
| **PLGA amount (g)** | 2.0 | 2.0 |
| **PLGA L/G ratio** | 50:50 | 75:25 |
| **PLGA M.W. (kDa)** | 38-54 | 76-115 |
| **Solvent amount (g)** | 20.0 | 20.0 |
| **PVA concentration (%, w/v)** | 2 | 2 |
| **PVA solution amount (mL)** | 150 | 150 |

### Experimental Example 3: Analysis of microsphere properties and drug encapsulation rate

The properties of the microspheres prepared by Example 41 (test microspheres 1 to 16) and Example 42 (comparative microspheres 1 and 2) were characterized by drug precipitation during manufacture, the morphology of lyophilized microspheres, and floating in the aqueous phase upon redispersion. Whether or not precipitation of the drug was confirmed through an optical microscope during manufacture, and the morphology of the lyophilized microspheres were observed by scanning electron microscopy. Whether or not floating of the microspheres in the aqueous phase was confirmed by redispersing the lyophilized microspheres in water. For the amount of drug encapsulated in the microspheres, 30 mg of microspheres were dissolved in 50 mL of acetonitrile, and the supernatant obtained by ultracentrifugation was analyzed by HPLC (high performance liquid chromatography). In addition, the appearance of a large amount of caspase inhibitor crystals precipitated during the preparation of microspheres in comparative microspheres 1 and 2 was confirmed by using an optical microscope.

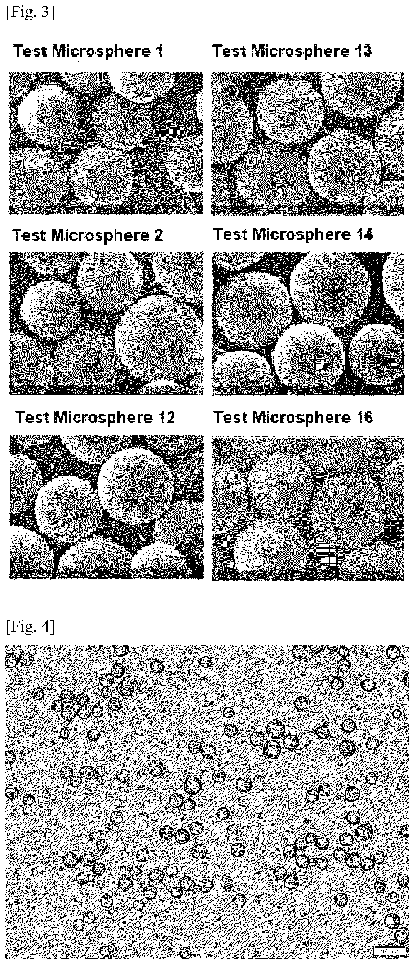

The results of observation or measurement of whether or not drug precipitation in the test microspheres 1, 2, 12 to 14 and 16, the morphology of the microspheres, whether or not microsphere floating, and drug encapsulation rate (%, w/w) are represented in Table 5 below. In addition, the morphology of these test microspheres were observed by scanning electron microscopy and represented in Figure 3. The microspheres had a diameter of about 50 µm, and all had good surfaces. In the test microsphere 2 of Figure 3, the substance appearing as needle-like is that remains in the form of drug crystals and can be removed by washing.

**[Table 5]**

| **Test microsphere No.** | **1** | **2** | **12** | **13** | **14** | **16** |
|---|---|---|---|---|---|---|
| **Drug precipitation** | None | None | None | None | Small amount | None |
| **Microsphere morphology** | Good | Good | Good | Good | Good | Good |
| **Microsphere floating** | None | None | None | None | None | None |
| **Drug encapsulation rate (%, w/w)** | 15.2 | 13.9 | 16.0 | 14.5 | 15.5 | 15.1 |

The results of observation or measurement of drug precipitation in the comparative microspheres 1 and 2, the morphology of the microspheres, whether or not microsphere floating, and drug encapsulation rate (%, w/w) are represented in Table 6 below. The comparative microspheres containing caspase inhibitor had good morphology and no microsphere floating phenomenon, but a large amount of drug was precipitated during the manufacturing process. The drug encapsulation rate was observed as about 8% in both comparative microspheres 1 and 2, and was confirmed to be about half the drug encapsulation rate of the test microspheres measured in Table 5 above. In addition, according to Figure 4, it was confirmed that a large amount of crystals of the caspase inhibitor were precipitated in the comparative microspheres 1 and 2 during the curing step.

**[Table 6]**

| **Comparative microsphere No.** | **1** | **2** |
|---|---|---|
| **Drug precipitation** | Large amount | Large amount |
| **Microsphere morphology** | Good | Good |
| **Microsphere floating** | None | None |
| **Drug encapsulation rate (%, w/w)** | 8.2 | 7.8 |

Consequently, it was confirmed that the drug encapsulation efficiency was not good when the microspheres were prepared using the caspase inhibitor, but the drug encapsulation efficiency was greatly improved when the microspheres were prepared using the caspase inhibitor prodrug.

### Example 43: Preparation of sustained-release microspheres for intra-articular administration using caspase inhibitor prodrug of Example 16

According to the composition denoted in Table 7 below, the test microspheres 17 to 24, which are drug sustained-release microspheres for intra-articular administration, encapsulated with the caspase inhibitor prodrug of Example 16 were prepared. The weight ratio of the prodrug compound and PLGA was weighed at 10, 13, 16 or 20% (w/w) as denoted in Table 7 below, and an organic solvent dichloromethane was added thereto and stirred to prepare the dispersed phase. The L/G ratio of the PLGA used was different in two ways: 50:50 (M.W. 38,000 - 54,000) and 75:25 (M.W. 76,000 - 115,000). For the continuous phase, 150 mL of 2% polyvinyl alcohol (M.W. 31,000 - 50,000, hydrolysis degree 87 - 89 %) was used, and an emulsion was prepared by a membrane emulsification method. The prepared emulsion was stirred overnight at room temperature to remove the solvent, washed with sterile purified water repeatedly, and then freeze-dried to prepare microspheres.

**[Table 7]**

| **Test microsphere No.** | **17** | **18** | **19** | **20** | **21** | **22** | **23** | **24** |
|---|---|---|---|---|---|---|---|---|
| **Caspase inhibitor prodrug** | Example 16 | Example 16 | Example 16 | Example 16 | Example 16 | Example 16 | Example 16 | Example 16 |
| **Prodrug amount (g)** | 0.2 | 0.26 | 0.32 | 0.4 | 0.2 | 0.26 | 0.32 | 0.4 |
| **PLGA amount (g)** | 2.0 | 2.0 | 2.0 | 2.0 | 2.0 | 2.0 | 2.0 | 2.0 |
| **PLGA L/G ratio** | 50:50 | 50:50 | 50:50 | 50:50 | 75:25 | 75:25 | 75:25 | 75:25 |
| **PLGA M.W. (kDa)** | 38-54 | 38-54 | 38-54 | 38-54 | 76-115 | 76-115 | 76-115 | 76-115 |
| **Pro drug/PLGA (%, w/w)** | 10 | 13 | 16 | 20 | 10 | 13 | 16 | 20 |
| **Solvent amount (g)** | 20.0 | 20.0 | 20.0 | 20.0 | 20.0 | 20.0 | 20.0 | 20.0 |
| **PVA concentration (%, w/v)** | 2 | 2 | 2 | 2 | 2 | 2 | 2 | 2 |
| **PVA solution amout (mL)** | 150 | 150 | 150 | 150 | 150 | 150 | 150 | 150 |

### Experimental Example 4: Analysis of physical stability and drug encapsulation rate of microspheres

The physical stability of the test microspheres 17 to 24 was judged by whether aggregation occurred by shaking for one day in a buffer solution, PBS (phosphate buffered saline, 37°C). Table 8 represents the results of analysis of the physical stability and drug encapsulation rate of the test microspheres 17 to 24. In the test microspheres 17 to 19, aggregation of microspheres in PBS was not observed within one day, but in the test microsphere 20, it was found. In addition, in the test microspheres 21 to 23, the phenomenon of aggregation of microspheres was not found in one day, but it was found in the test microsphere 24. Because it was confirmed that most of the loading amount of the prodrug of Example 16 was encapsulated during the preparation of microspheres, the encapsulation rate was not separately measured in this test.

**[Table 8]**

| | | | | | | | | |
|---|---|---|---|---|---|---|---|---|
| **Test microsphere No.** | **17** | **18** | **19** | **20** | **21** | **22** | **23** | **24** |
| **Prodrug/PLGA (%, w/w)** | 10 | 13 | 16 | 20 | 10 | 13 | 16 | 20 |
| **Microsphere aggregation** | None | None | None | Serious | None | None | None | Serious |

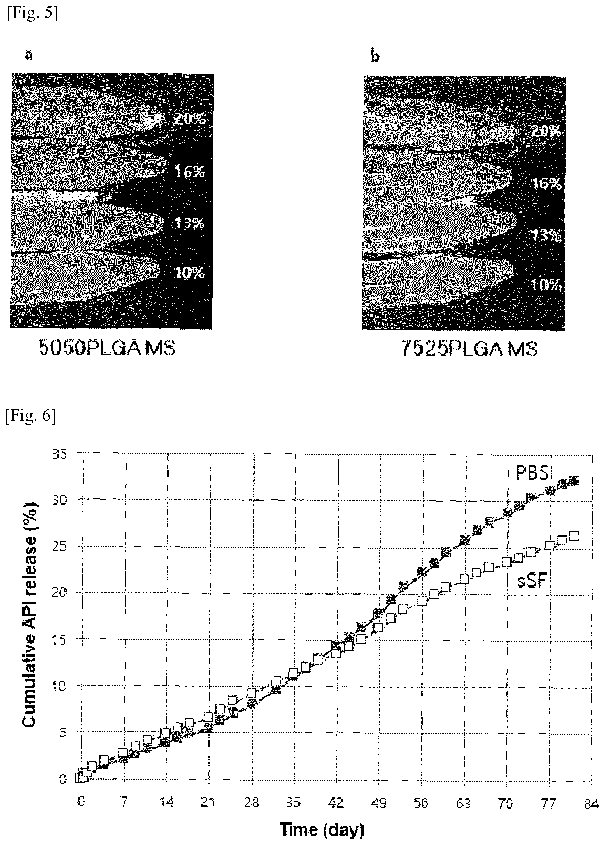

The state in which the test microspheres 17 to 24 were dispersed in PBS after one day is represented in Figure 5. According to Figure 5a, it was confirmed that the microspheres encapsulated with 10%, 13% and 16% (theoretical encapsulation rate) of the prodrug of Example 16, respectively, were well dispersed in PBS and particles were not visible, but the microspheres encapsulated with 20% (theoretical encapsulation rate) the prodrug of Example 16 were aggregated and agglomerated. According to Figure 5b, it was confirmed that the microspheres encapsulated in 10%, 13% and 16% (theoretical encapsulation rate) of the prodrug of Example 16, respectively, were well dispersed in PBS and particles were not visible, but the microspheres encapsulated with 20% (theoretical encapsulation rate) of the prodrug of Example 16 were aggregated and agglomerated.

Consequently, it was confirmed that the microspheres were aggregated in PBS within one day when the microspheres containing 20% or more of the prodrug of Example 16 were prepared.

### Experimental Example 5: In vitro dissolution test of microspheres containing prodrug of Example 2

An *in vitro* dissolution test of the microspheres prepared by encapsulating the prodrug of Example 2 according to the test microsphere 2 was carried out. More specifically, the microspheres were shaken in PBS buffer (37°C), the eluate was collected and filtered at specific times, and the amount of drug released was analyzed by HPLC. Becasue the prodrug is converted to the parent drug, caspase inhibitor, by hydrolysis in aqueous solution, the amount of the released drug was confirmed through the amount of caspase inhibitor measured by HPLC.

In addition, the same experiment was repeated in PBS containing 1% of hyaluronic acid and 2.5% of bovine serum albumin-which simulated synovial fluid, and it was checked whether the dissolution pattern was changed in simulated synovial fluid (sSF).

The *in vitro* dissolution graph of PLGA microspheres encapsulated with the prodrug of Example 2 is represented in Figure 6. As a result of the experiment, it was confirmed that the dissolution of the caspase inhibitor continued for about 12 weeks, and no difference in dissolution patterns was found in the two dissolution test solutions of PBS and sSF. In conclusion, because no difference was found in dissolution patterns in PBS and sSF, it was confirmed that there was no need to use sSF in a subsequent dissolution test.

### Experimental Example 6: In vitro dissolution test of microspheres containing prodrug of Example 16

An *in vitro* dissolution test of the microspheres prepared by encapsulating the prodrug of Example 16 according to the test microspheres 18 and 22 was carried out. More specifically, the microspheres were shaken in PBS buffer (37°C), the eluate was collected and filtered at specific times, and the amount of the drug released was analyzed by HPLC. Because the prodrug is converted to the parent drug, caspase inhibitor, by hydrolysis in aqueous solution, the amount of the drug released was confirmed through the amount of caspase inhibitor measured by HPLC. In addition, the properties of the microspheres that change with the progress of hydrolysis were confirmed by scanning electron microscopy. The molecular weight of PLGA, which changes as hydrolysis proceeds, was measured using GPC (gel permeation chromatograhy).

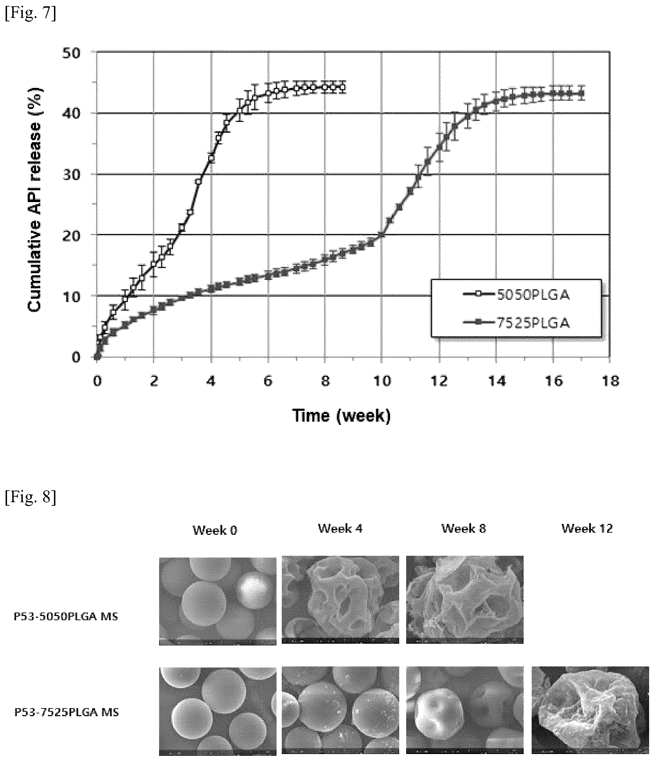

The *in vitro* dissolution graph of PLGA microspheres according to test microspheres 18 and 22 is represented in Figure 7. The dissolution of the test microsphere 18 with a PLGA L/G ratio of 50:50 continued for about 6 weeks, and that of the test microsphere 22 with an L/G ratio of 75:25 continued for about 14 weeks. Both types of the microspheres showed a pattern in which the dissolution continued with a gentle slope after the initial burst, and the amount of dissolution rapidly increased in the middle.

The photographs observing the shape of the microspheres during the dissolution test is represented in Figure 8. The test microspheres 18 (5050 PLGA) had more pores at about week 4, and at week 8 the microspheres were swollen and enlarged with larger pores. In the test microsphere 22 (7525 PLGA), pore formation was observed gradually at week 8, and PLGA swelled and the shape of the sphere collapsed at week 12.

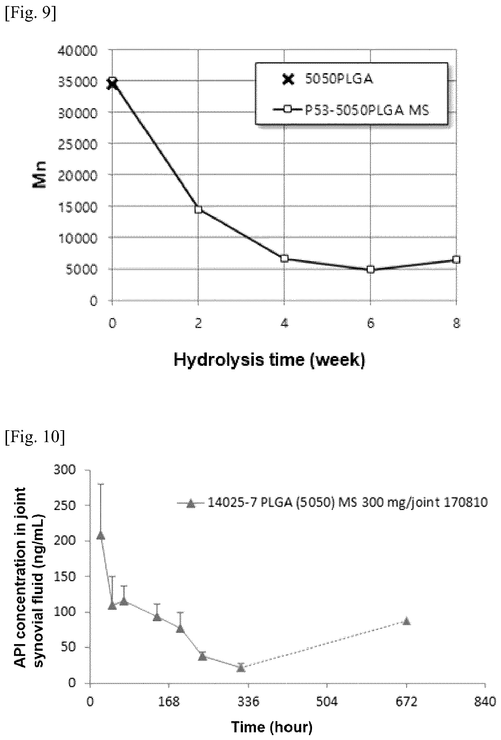

The graph measuring the molecular weight change of the test microsphere 18 (5050 PLGA) during the dissolution test is represented in Figure 9. As the dissolution test started, the molecular weight decreased rapidly, and at week 4 the molecular weight dropped to about 1/5 of the original one and maintained a similar level thereafter.

As a result of the dissolution test, the drug on the surface of the microspheres was rapidly eluted as PLGA was decomposed by hydrolysis from the beginning of dissolution, and it was analyzed that when PLGA was further decomposed, pores were formed and the drug was actively released and diffused at a specific point in time. In addition, it was confirmed that a more delayed release pattern was observed when using PLGA (7525PLGA) having an L/G ratio of 75:25 than when using PLGA (5050PLGA) having an L/G ratio of 50:50, and the morphology were changed and the molecular weight decreased by hydrolysis.

### Experimental Example 7: Pharmacokinetic (PK) test of microspheres containing prodrug of Example 2

The PK test was carried out in dogs using microspheres prepared by encapsulating the prodrug of Example 2 according to the test microsphere 2. Microspheres at a concentration of 300 mg/ml were administered to the joint cavity, and joint synovial fluid was collected at a specific time point to measure the concentration of caspase inhibitor, and the results are represented in Figure 10.

As a result of measuring caspase inhibitors in the joint cavity, the initial increase occurred significantly after 24 hours, and the concentration gradually decreased thereafter. It was expected to last up to 2 weeks, but it was confirmed that the test microsphere 2 (5050PLGA) encapsulated with the prodrug of Example 2 releases the caspase inhibitor continuously for about 4 weeks since the concentration was slightly increased when measured at 4 weeks later. In conclusion, it was confirmed that the caspase inhibitor was continuously released for about 4 weeks in the test microsphere 2 encapsulated with the prodrug of Example 2.

## Claims

1. A compound of the following Formula 1, or a pharmaceutically acceptable salt or isomer thereof: 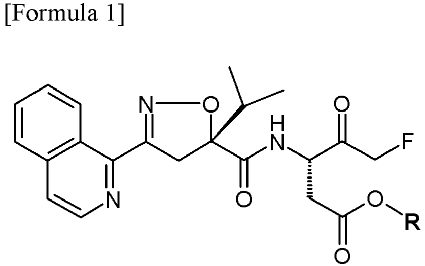 wherein
R represents alkyl, haloalkyl, cycloalkyl, cycloalkylalkyl, aryl, arylalkyl, heteroaryl, heteroarylalkyl, alkoxy or alkoxyalkyl, wherein the heteroaryl includes one or more heteroatoms selected from N, O and S;
wherein the alkyl, haloalkyl, cycloalkyl, cycloalkylalkyl, aryl, arylalkyl, heteroaryl, heteroarylalkyl or alkoxyalkyl is optionally substituted, and the substituent may be one or more selected from alkyl, halo, haloalkyl, cycloalkyl, hydroxy, acyl, amino, alkoxy, carboalkoxy, oxo, carboxy, carboxyamino, cyano, nitro, thiol, aryloxy, sulfoxy and guanido group;
provided that R is not tert-butyl.

2. The compound, or a pharmaceutically acceptable salt or isomer thereof according to Claim 1, wherein
R represents C₁-₂₀ alkyl, C₃-₁₀ cycloalkyl, C₃-₁₀ cycloalkyl-C₁-₆ alkyl, C₆-C₁₀ aryl, C₆-C₁₀ aryl-C₁-₆ alkyl, 3- to 10-membered heteroaryl, 3- to 10-membered heteroaryl-C₁-₆ alkyl, halo-C₁-₆ alkyl or C₁-₆ alkoxy-C₁-₆ alkyl, wherein the heteroaryl includes 1 to 4 heteroatoms selected from N, O and S.

3. The compound, or a pharmaceutically acceptable salt or isomer thereof according to Claim 1, wherein
R represents C₁-₁₈ alkyl, C₃-₆ cycloalkyl, C₃-₆ cycloalkyl-C₁-₃ alkyl, C₆-C₁₀ aryl, C₆-C₁₀ aryl-C₁-₃ alkyl, 4- to 6-membered heteroaryl-C₁-₃ alkyl, halo-C₁-₃ alkyl or C₁-₃ alkoxy-C₁-₃ alkyl, wherein the heteroaryl includes 1 or 2 heteroatoms selected from N, O and S, and the substituent is alkyl, halo, alkoxy or oxo.

4. The compound according to Claim 1, wherein the compound is selected from the following group:
methyl (S)-5-fluoro-3-((R)-5-isopropyl-3-(isoquinolin-1-yl)-4,5-dihydroisoxazole-5-carboxamido)-4-oxopentanoate;
ethyl (S)-5-fluoro-3-((R)-5-isopropyl-3-(isoquinolin-1-yl)-4,5-dihydroisoxazole-5-carboxamido)-4-oxopentanoate;
propyl (S)-5-fluoro-3-((R)-5-isopropyl-3-(isoquinolin-1-yl)-4,5-dihydroisoxazole-5-carboxamido)-4-oxopentanoate;
butyl (S)-5-fluoro-3-((R)-5-isopropyl-3-(isoquinolin-1-yl)-4,5-dihydroisoxazole-5-carboxamido)-4-oxopentanoate;
isobutyl (S)-5-fluoro-3-((R)-5-isopropyl-3-(isoquinolin-1-yl)-4,5-dihydroisoxazole-5-carboxamido)-4-oxopentanoate;
isopentyl (S)-5-fluoro-3-((R)-5-isopropyl-3-(isoquinolin-1-yl)-4,5-dihydroisoxazole-5-carboxamido)-4-oxopentanoate;
pentyl (S)-5-fluoro-3-((R)-5-isopropyl-3-(isoquinolin-1-yl)-4,5-dihydroisoxazole-5-carboxamido)-4-oxopentanoate;
hexyl (S)-5-fluoro-3-((R)-5-isopropyl-3-(isoquinolin-1-yl)-4,5-dihydroisoxazole-5-carboxamido)-4-oxopentanoate;
heptyl (S)-5-fluoro-3-((R)-5-isopropyl-3-(isoquinolin-1-yl)-4,5-dihydroisoxazole-5-carboxamido)-4-oxopentanoate;
octyl (S)-5-fluoro-3-((R)-5-isopropyl-3-(isoquinolin-1-yl)-4,5-dihydroisoxazole-5-carboxamido)-4-oxopentanoate;
decyl (S)-5-fluoro-3-((R)-5-isopropyl-3-(isoquinolin-1-yl)-4,5-dihydroisoxazole-5-carboxamido)-4-oxopentanoate;
dodecyl (S)-5-fluoro-3-((R)-5-isopropyl-3-(isoquinolin-1-yl)-4,5-dihydroisoxazole-5-carboxamido)-4-oxopentanoate;
pentadecyl (S)-5-fluoro-3-((R)-5-isopropyl-3-(isoquinolin-1-yl)-4,5-dihydroisoxazole-5-carboxamido)-4-oxopentanoate;
octadecyl (S)-5-fluoro-3-((R)-5-isopropyl-3-(isoquinolin-1-yl)-4,5-dihydroisoxazole-5-carboxamido)-4-oxopentanoate;
(9E,12E)-octadeca-9,12-dien-1-yl (S)-5-fluoro-3-((R)-5-isopropyl-3-(isoquinolin-1-yl)-4,5-dihydroisoxazole-5-carboxamido)-4-oxopentanoate;
cyclopropylmethyl (S)-5-fluoro-3-((R)-5-isopropyl-3-(isoquinolin-1-yl)-4,5-dihydroisoxazole-5-carboxamido)-4-oxopentanoate;
cyclobutylmethyl (S)-5-fluoro-3-((R)-5-isopropyl-3-(isoquinolin-1-yl)-4,5-dihydroisoxazole-5-carboxamido)-4-oxopentanoate;
cyclopentylmethyl (S)-5-fluoro-3-((R)-5-isopropyl-3-(isoquinolin-1-yl)-4,5-dihydroisoxazole-5-carboxamido)-4-oxopentanoate;
allyl (S)-5-fluoro-3-((R)-5-isopropyl-3-(isoquinolin-1-yl)-4,5-dihydroisoxazole-5-carboxamido)-4-oxopentanoate;
isopropyl (S)-5-fluoro-3-((R)-5-isopropyl-3-(isoquinolin-1-yl)-4,5-dihydroisoxazole-5-carboxamido)-4-oxopentanoate;
penta-3-yl (S)-5-fluoro-3-((R)-5-isopropyl-3-(isoquinolin-1-yl)-4,5 dihydroisoxazole-5-carboxamido)-4-oxopentanoate;
sec-butyl (S)-5-fluoro-3-((R)-5-isopropyl-3-(isoquinolin-1-yl)-4,5-dihydroisoxazole-5-carboxamido)-4-oxopentanoate;
pentan-2-yl (S)-5-fluoro-3-((R)-5-isopropyl-3-(isoquinolin-1-yl)-4,5-dihydroisoxazole-5-carboxamido)-4-oxopentanoate;
heptan-2-yl (S)-5-fluoro-3-((R)-5-isopropyl-3-(isoquinolin-1-yl)-4,5-dihydroisoxazole-5-carboxamido)-4-oxopentanoate;
cyclopentyl (S)-5-fluoro-3-((R)-5-isopropyl-3-(isoquinolin-1-yl)-4,5-dihydroisoxazole-5-carboxamido)-4-oxopentanoate;
cyclohexyl (S)-5-fluoro-3-((R)-5-isopropyl-3-(isoquinolin-1-yl)-4,5-dihydroisoxazole-5-carboxamido)-4-oxopentanoate
benzyl (S)-5-fluoro-3-((R)-5-isopropyl-3-(isoquinolin-1-yl)-4,5-dihydroisoxazole-5-carboxamido)-4-oxopentanoate;
(5-methyl-2-oxo-1,3-dioxol-4-yl)methyl (S)-5-fluoro-3-((R)-5-isopropyl-3-(isoquinolin-1-yl)-4,5-dihydroisoxazole-5-carboxamido)-4-oxopentanoate;
2-methoxyphenyl (S)-5-fluoro-3-((R)-5-isopropyl-3-(isoquinolin-1-yl)-4,5-dihydroisoxazole-5-carboxamido)-4-oxopentanoate;
2,3-dihydro-1*H*-inden-5-yl (S)-5-fluoro-3-((R)-5-isopropyl-3-(isoquinolin-1-yl)-4,5-dihydroisoxazole-5-carboxamido)-4-oxopentanoate;
naphthalen-1-yl (S)-5-fluoro-3-((R)-5-isopropyl-3-(isoquinolin-1-yl)-4,5-dihydroisoxazole-5-carboxamido)-4-oxopentanoate;
phenyl (S)-5-fluoro-3-((R)-5-isopropyl-3-(isoquinolin-1-yl)-4,5-dihydroisoxazole-5-carboxamido)-4-oxopentanoate;
naphthalen-1-ylmethyl (S)-5-fluoro-3-((R)-5-isopropyl-3-(isoquinolin-1-yl)-4,5-dihydroisoxazole-5-carboxamido)-4-oxopentanoate;
2,2,2-trifluoroethyl (S)-5-fluoro-3-((R)-5-isopropyl-3-(isoquinolin-1-yl)-4,5-dihydroisoxazole-5-carboxamido)-4-oxopentanoate;
2-methoxyethyl (S)-5-fluoro-3-((R)-5-isopropyl-3-(isoquinolin-1-yl)-4,5-dihydroisoxazole-5-carboxamido)-4-oxopentanoate;
2-fluoroethyl (S)-5-fluoro-3-((R)-5-isopropyl-3-(isoquinolin-1-yl)-4,5-dihydroisoxazole-5-carboxamido)-4-oxopentanoate;
neopentyl (S)-5-fluoro-3-((R)-5-isopropyl-3-(isoquinolin-1-yl)-4,5-dihydroisoxazole-5-carboxamido)-4-oxopentanoate;
thiophen-2-ylmethyl (S)-5-fluoro-3-((R)-5-isopropyl-3-(isoquinolin-1-yl)-4,5-dihydroisoxazole-5-carboxamido)-4-oxopentanoate;
thiophen-3-ylmethyl (S)-5-fluoro-3-((R)-5-isopropyl-3-(isoquinolin-1-yl)-4,5-dihydroisoxazole-5-carboxamido)-4-oxopentanoate; and
furan-3-ylmethyl (S)-5-fluoro-3-((R)-5-isopropyl-3-(isoquinolin-1-yl)-4,5-dihydroisoxazole-5-carboxamido)-4-oxopentanoate.

5. The compound according to Claim 1 which as a prodrug of caspase inhibitor, or a pharmaceutically acceptable salt or isomer thereof.

6. A pharmaceutical composition for the prevention or treatment of inflammation or apoptosis comprising the compound of Formula 1, or a pharmaceutically acceptable salt or isomer thereof as defined in any one of Claims 1 to 5 as an active ingredient, together with a pharmaceutically acceptable carrier.

7. The pharmaceutical composition according to Claim 6, which is formulated in oral dosage form, injection form or patch form.

8. The pharmaceutical composition according to Claim 6, which comprises a microsphere comprising the compound of Formula 1, or a pharmaceutically acceptable salt or isomer, and a biocompatible polymer.

9. The pharmaceutical composition according to Claim 8, wherein the biocompatible polymer is a polylactide-glycolide copolymer having a molar ratio of lactide to glycolide of 10:90 to 90:10.

10. The pharmaceutical composition according to Claim 8, wherein the weight ratio of the compound of Formula 1, or a pharmaceutically acceptable salt or isomer thereof, and the biocompatible polymer in the microsphere is 1:100 to 70:100.

11. A pharmaceutical composition for the prevention or treatment of a disease selected from apoptosis-associated diseases, inflammatory diseases, osteoarthritis, rheumatoid arthritis, degenerative arthritis and destructive bone disorders, comprising the compound of Formula 1, or a pharmaceutically acceptable salt or isomer thereof as defined in any one of Claims 1 to 5 as an active ingredient, together with a pharmaceutically acceptable carrier.
